# EUROPEAN PATENT SPECIFICATION

(11) **EP 2 952 252 B1**
(45) Date of publication and mention of the grant of the patent: **22.05.2019**
(21) Application number: 14746478.8
(22) Date of filing: 31.01.2014
(51) Int. Cl.: B01J 23/66, B01J 35/08, B01J 37/04, B01J 37/08, C07C 1/247, C07C 15/46, C07C 29/141, C07C 29/145, C07C 33/02, C07C 33/14, C07C 33/20, C07C 33/22, C07C 33/48, C07C 35/44, C07C 41/26, C07C 43/23, C07B 61/00

(54) **SELECTIVE HYDROGENATION CATALYST, PRODUCTION METHOD FOR SELECTIVE HYDROGENATION CATALYST, AND SELECTIVE HYDROGENATION METHOD**
SELEKTIVER HYDRIERUNGSKATALYSATOR, VERFAHREN ZUR HERSTELLUNG EINES SELEKTIVEN HYDRIERUNGSKATALYSATORS UND SELEKTIVES HYDRIERUNGSVERFAHREN
CATALYSEUR D'HYDROGÉNATION SÉLECTIVE, PROCÉDÉ DE PRODUCTION DU CATALYSEUR D'HYDROGÉNATION SÉLECTIVE ET PROCÉDÉ D'HYDROGÉNATION SÉLECTIVE

(30) Priority: 31.01.2013 JP 2013017005
(43) Date of publication of application: 09.12.2015
(73) Proprietor: Osaka University, Suita-shi, Osaka 565-0871 (JP); N.E. Chemcat Corporation, Tokyo 105-6124 (JP)
(72) Inventor: KANEDA, Kiyotomi, Suita-shi Osaka 565-0871 (JP); TAKAGI, Yukio, Numazu-shi Shizuoka 410-0314 (JP)
(74) Representative: von Füner, Nicolai
(86) International application number: PCT/JP2014/052360
(87) International publication number: WO 2014/119766

(56) References cited:
- MOTOSHI MATOBA ET AL.: 'Sentakuteki Kangen Hanno o Kano ni suru Core-Shell-gata Gin Nano Ryushi Shokubai no Kaihatsu' DAI 110 KAI SHOKUBAI TORONKAI TORONKAI A YOKOSHU 14 September 2012, page 417, XP008177446
- TAKATO MITSUDOME ET AL.: 'Bunshijo Suiso Mochiita Kosentakuteki Kangen Hanno o Kano to suru Shinki Core-Shell-gata Gin Nano Ryushi Shokubai no Kaihatsu' DAI 108 KAI SHOKUBAI TORONKAI TORONKAI A YOKOSHU 13 September 2011, pages 120 - 121, XP008177448
- MITSUDOME TAKAO ET AL.: 'Design of a Silver- Cerium Dioxide Core-Shell Nanocomposite Catalyst for Chemoselective Reduction Reactions' ANGEW. CHEM. INT. ED. 2012, pages 136 - 139, XP055211953
- SHIMIZU KEN-ICHI ET AL.: 'Size- and support- dependent silver cluster catalysis for chemoselective hydrogenation of nitroaromatics' JOURNAL OF CATALYSIS vol. 270, no. 1, 22 March 2010, pages 86 - 94, XP026916184

## Description

### TECHNICAL FIELD

The present invention relates to a selective hydrogenation catalyst exhibiting a superior reactivity and selectivity to the oxygen-containing polar functional group in a compound containing a hydrogenation reaction site and an oxygen-containing polar functional group; a production method of such selective hydrogenation catalyst; and a method of performing selective hydrogenation using the selective hydrogenation catalyst. Specifically, the present invention relates to a selective hydrogenation catalyst exhibiting a superior reactivity and selectivity to the oxygen-containing polar functional group in a compound containing a hydrogenation reaction site such as a carbon-carbon double bond, and an oxygen-containing polar functional group such as an epoxide bond; a method for producing the selective hydrogenation catalyst; and a method of selectively hydrogenating the compound using the selective hydrogenation catalyst.

### BACKGROUND ART

Up to now, a variety of studies have been made on the method of selectively hydrogenating oxygen atoms in an oxygen-containing polar functional group in a compound containing a carbon-carbon double bond and the oxygen-containing polar functional group. For example, non-patent document 1 proposes a selective hydrogenation method for selectively hydrogenating a nitro group in an aromatic compound containing a carbon-carbon double bond and a nitro group.

This selective hydrogenation method is aimed to selectively hydrogenate and thus transform a nitro group of aromatic compounds into an amino group by bringing the aromatic compounds, containing a carbon-carbon double bond and a nitro group, into contact with a hydrogen gas under the presence of a silver component-cerium oxide complex that comprises silver component particles and a cerium oxide supported on the surfaces of the silver component particles in a liquid phase containing an organic solvent.

The selective hydrogenation method according to non-patent document 1 utilizes molecular hydrogen as a source of hydrogen for hydrogenating the nitro groups, eliminating the need for the use of any carbon monoxides or alcohols, which have been conventionally required for selectively hydrogenating nitro groups.

Accordingly, there can be avoided any use of highly toxic carbon monoxides by virtue of the above mentioned selective hydrogenation method, which exhibits superior reactivity and selectivity as a superior selective hydrogenation method. Further, since the above mentioned selective hydrogenation method is a method in which a nitro group of the aromatic compound, containing a carbon-carbon double bond and the nitro group, is selectively hydrogenated under the presence of the silver component-cerium oxide complex that comprises silver component particles and the cerium oxide supported on the surfaces of the silver component particles, the method is advantageous in that no operation is required for separating the aromatic compound containing the amino group i.e. a desired product from its by-products after the completion of the hydrogenation reaction.

That is, as for the methods for selectively hydrogenating the oxygen atoms of the nitro group of a compound containing a carbon-carbon double bond and the nitro group, the molecular hydrogen, used for the hydrogenation reaction, is advantageous as a hydrogen source for the hydrogenation reaction in that the molecular hydrogen, involving no production of any products, is free from toxicity problems, thus leading to a significant reduction in environmental burdens. The applicants of the present invention provide the following literature as a literature describing the above mentioned publicly known invention.

### PRIOR ART DOCUMENT

### Non-patent document

Non-patent document 1: The proceedings of 108-th catalyst symposium, Catalysis Society of Japan, September 13, 2011, page 120 to 121 .

Mitsudome Takao et al. ("Design of a Silver-Cerium Dioxide Core-Shell Nanocomposite Catalyst for Chemoselective Reduction Reactions", Angew. Chem. Int. Ed., 2012, pages 136-139) discloses an AgNPs@CeO₂ catalyst with AgNPs being supported on CeO₂. Further, said document discloses selective reduction reactions wherein nitrostyrenes are reduced to aminostyrenes with the help of said AgNPs@CeO₂ catalyst in the presence of hydrogen.

### SUMMARY OF THE INVENTION

Disclosed are a selective hydrogenation catalyst as set forth in independent claim 1, a selective hydrogenation method as set forth in independent claim 5 and a production method of a selective hydrogenation catalyst as set forth in independent claim 6.

### Problem to be solved by the invention

However, the above mentioned selective hydrogenation method has problems that targeted substrates for the selective hydrogenation reaction are limited only to compounds containing a carbon-carbon double bond and a nitro group and that said compounds have extremely limited variety of oxygen-containing groups and hydrogenation reaction sites; that is, the range to which the compounds can be applied is extremely narrow.
Not only that, the above mentioned selective hydrogenation method has a problem of hydrogenating not only oxygen-containing polar functional group but also hydrogenation reaction site, thus not being able to selectively hydrogenate the oxygen-containing polar functional group even in a case where the method is applied to the selective hydrogenation method targeted for oxygen-containing polar functional group in a compound containing a hydrogenation reaction site such as a carbon-carbon double bond, and an oxygen-containing polar functional group such as an epoxide bond by using a catalyst of the silver component-cerium oxide complex containing silver component particles and the cerium oxide supported on the surfaces of the silver component particles, the catalyst being used in the above mentioned selective hydrogenation method. Moreover, it has been extremely difficult to achieve both
superior reactivity and selectivity at the same time even in a case where the above mentioned silver component-cerium oxide complex is used as a selective hydrogenation catalyst for performing selective hydrogenation reaction with respect to the oxygen-containing polar functional group of the above mentioned compound.

Under these circumstances, there have been such demands of selective hydrogenation catalyst, production method of the selective hydrogenation catalyst, and selective hydrogenation method capable of hydrogenating only the oxygen-containing polar functional group with a high conversion rate and a high selectivity in compounds containing: not only carbon-carbon double bond but also other hydrogenation reaction sites; and not only nitro group but also other oxygen-containing polar functional groups.

The invention is proposed in consideration of such problems, and an object thereof is to provide a selective hydrogenation catalyst capable of selectively hydrogenating the oxygen-containing polar functional group with a high conversion rate and a high selectivity in compounds containing hydrogenation reaction site and oxygen-containing polar functional group. The present invention also provides a method for selectively hydrogenating the above mentioned compounds, using the selective hydrogenation catalyst. Further, the present invention also provides various kinds of hydrogenated compound in which the oxygen of the oxygen-containing polar functional groups in, e.g. unsaturated alcohols, amino compounds, and olefin compound, are hydrogenated by the above described selective hydrogenation method to provide the hydrogenated compounds. According to another aspect of the present invention, the object of the present invention is to provide a method for producing the selective hydrogenation catalyst capable of firmly supporting catalytic components on the metal oxide support without using any particular binders, and securing the safety in production process.

### Means to solve the problem

The inventors of the present invention have studied earnestly to find the viability of hydrogenating only oxygen-containing polar functional group with a significantly high conversion rate and a significantly high selectivity in a compound containing hydrogenation reaction site such as carbon-carbon double bond and oxygen-containing polar functional group, using a selective hydrogenation catalyst with silver component-cerium oxide supported on the metal oxide support, the silver component-cerium oxide containing silver component particles and the cerium oxide supported on the surfaces of the silver component particles to perform the hydrogenation reaction, and finally made the discovery thereof. More specifically, the present inventions comprise the following technical matters.
(1) A selective hydrogenation catalyst comprising:
   a metal oxide support; and
   a silver component-cerium oxide complex that is supported on said metal oxide support and includes silver component particles as a core and cerium oxide particles as a shell layer supported on the surfaces of said silver component particles.
(2) The selective hydrogenation catalyst according to (1), wherein said silver component-cerium oxide complex exhibits a molar ratio of 0.25 to 3.0 in terms of metallic silver (Ag) / cerium oxide (CeO2), said molar ratio being a molar ratio of metallic silver in said silver component particles to said cerium oxide.
(3) The selective hydrogenation catalyst according to (1) or (2) wherein said metal oxide support is a cerium-containing oxide.
(4) The selective hydrogenation catalyst according to any one of (1) to (3), wherein said silver component-cerium oxide complex is formed into the shape of a true sphere.
(5) A selective hydrogenation method for hydrogenating a compound having a hydrogenation reaction site and an oxygen-containing polar functional group, comprising a step of bringing said compound into contact with a hydrogen gas in a liquid phase under the presence of said catalyst of any one of (1) to (4).
(6) A production method of a selective hydrogenation catalyst comprising a metal oxide support and a silver component-cerium oxide complex that is supported on said metal oxide support and includes silver component particles as a core and cerium oxide particles as a shell layer supported on the surfaces of said silver component particles, comprising:
   a step of preparing a mixture by adding said metal oxide support to a liquid mixture of a reverse micelle containing said silver component particles and a reverse micelle containing said cerium oxide;
   a step of drying said mixture to form a catalyst precursor with said silver component-cerium oxide complex being supported on said metal oxide support; and
   a step of calcining said catalyst precursor.
(7) A production method of a selective hydrogenation catalyst comprising a metal oxide support and a silver component-cerium oxide complex that is supported on said metal oxide support and includes silver component particles as a core and cerium oxide particles as a shell layer supported on the surfaces of said silver component particles, comprising:
   a (i) step of preparing a raw material solution containing a mixed salt of a silver salt and a cerium salt;
   a (ii) step of forming on said metal oxide support coprecipitation mixed particles of a silver compound and a cerium compound, by mixing said raw material solution and said metal oxide support under the presence of a coprecipitation agent acting on both silver and cerium ions;
   a (iii) step of forming a catalyst precursor by adding a cerium salt to said coprecipitation mixed particles on said metal oxide support under the presence of said coprecipitation agent, and then allowing a precipitate of a cerium compound to be supported on said coprecipitation mixed particles; and
   a (iv) step of calcining said catalyst precursor.
(8) The selective hydrogenation catalyst production method according to (7), wherein said silver component is at least one of metal silver or silver oxide, and said cerium oxide is cerium dioxide.
(9) The selective hydrogenation catalyst production method according to (7), wherein said coprecipitation mixed particles in said step (iii) are mixed particles of silver compound particles and cerium compound particles that are both hardly soluble to a solvent of said raw material solution.
(10) The selective hydrogenation catalyst production method according to any one of (7) to (9), wherein said coprecipitation agent is an electrolyte containing negative ions capable of generating a hardly-soluble salt or a hardly-soluble compound with metal positive ions of any of silver ions and cerium ions.
(11) The selective hydrogenation catalyst production method according to any one of (7) to (10), wherein a suspension containing said metal oxide support is a suspension with cerium dioxide being suspended in a coprecipitation agent water solution.
(12) The selective hydrogenation catalyst production method according to any one of (8) to (11), wherein said silver salt and said cerium salt in said raw material solution have common negative ions.
(13) The selective hydrogenation catalyst production method according to (11), wherein a cerium ion precipitation in said step (iii) is coprecipitated on said metal oxide support.
(14) The selective hydrogenation catalyst production method according to any one of (7) to (13), wherein a measured value of a specific surface area of said metal oxide support is 0.5 to 180 m²/g, when measured through a BET method.

### Effects of the invention

According to the present invention, provided is a selective hydrogenation catalyst capable of selectively hydrogenating oxygen-containing polar functional group with a high conversion rate and a high selectivity in compounds containing hydrogenation reaction site such as carbon-carbon double bond, and oxygen-containing polar functional group such as epoxy bond. Further, according to the present invention, provided is a selective hydrogenation method using such catalyst.

According to the present invention, provided is a method for producing the selective hydrogenation catalyst capable of firmly supporting silver component-cerium oxide complex on the metal oxide support without using any particular binders. Further according to the present invention, provided is a production method of a selective hydrogenation catalyst, suitable for mass-producing the products, without
producing any explosive fulminating silver in the production process so as to ensure the safety thereof.

### BRIEF DESCRIPTION OF THE DRAWINGS

FIG.1 is an entire STEM (Scanning Transmission Electron Microscope) image of a selective hydrogenation catalyst [AgNPs@CeO₂-D] of the present invention.
FIG.2 is a pair of comparative views of: an image of AgNPs@CeO₂ on a support of selective hydrogenation catalyst [AgNPs@CeO₂-D] of the present invention, the image being obtained though mapping Ag and Ce elements respectively using High-Angle Annular Dark-Field Scanning Transmission Electron Microscopy (HAADF-STEM) and then superimposing the mapped images (Left); and schematic diagram of AgNPs@CeO₂ on the (CeO₂) support of [AgNPs@CeO₂-D] according to the present invention (Right).
FIG.3 is an image of the silver component-cerium oxide complex catalyst obtained through production example 2, the image being obtained using Field Emission-Scanning Electron Microscope (FE-SEM).
FIG.4 is a pair of images in which the image on the left is an image showing a silver component-cerium oxide complex catalyst obtained through production example 2, the image being obtained using Transmission Electron Microscope (TEM), and the image on the right is a schematic diagram for explaining the image on the left.
FIG.5 is an analysis chart showing analyzed results of composition distribution data of the components of the silver component-cerium oxide complex catalyst obtained through production example 2, the data being obtained by using Scanning Transmission Electron Microscope - Energy Dispersive X-ray Spectrometry (STEM-EDS).
FIG.6 is a schematic view showing a step for forming a cerium salt-supporting mixed particles in accordance with a selective hydrogenation catalyst production method using coprecipitation technique.
FIG.7 is an image of a silver component-cerium oxide complex catalyst obtained through production example 3, the image being obtained using Field Emission-Scanning Electron Microscope (FE-SEM).

### MODE FOR CARRYING OUT THE INVENTION

Described in detail hereunder are modes, especially various preferable modes for carrying out the present invention.

### <Selective hydrogenation catalyst>

A selective hydrogenation catalyst of the present invention comprises a metal oxide support and a silver component-cerium oxide complex supported thereon. Particularly, the silver component-cerium oxide complex contains silver component particles and a cerium oxide supported on the surfaces of the silver component particles. The selective hydrogenation catalyst of the present invention is described hereunder in terms of its components which are the silver component-cerium oxide complex and the metal oxide support. Here, the silver component-cerium oxide complex is obtained by employing silver nanoparticles (Ag Nano Particles) and/or silver oxide nanoparticles as cores, and then covering the same with a cerium oxide (mainly CeO₂, others include Ce₂O₃ etc.). Therefore, such silver component-cerium oxide complex is referred to as AgNPs@CeO₂ hereunder. Further, with regard to the selective hydrogenation catalyst, the silver component-cerium oxide complex is supported on the metal oxide support, and the silver component-cerium oxide complex is therefore dispersed (Dispersion), thus allowing the selective hydrogenation catalyst to be expressed as AgNPs@CeO₂-D hereunder.

### (Silver component-cerium oxide complex "AgNPs@CeO₂")

The silver component-cerium oxide complex "AgNPs@CeO₂" as a catalyst component used in the selective hydrogenation catalyst "AgNPs@CeO₂-D" of the present invention, is a complex with cerium oxide particles being supported on the surfaces of the silver component particles. Particularly, a desirable configuration of such silver component-cerium oxide complex "AgNPs@CeO₂" is that where the silver component particles serve as the cores, and the cerium oxide particles are supported on the surfaces of such silver component particles. Here, the silver component is at least one of metal silver or silver oxide; preferably metal silver or a mixture of metal silver and silver oxide; more preferably metal silver. Further, the cerium oxide is at least one of cerium dioxide (CeO₂) or cerium (III) oxide (Ce₂O₃); preferably cerium dioxide (CeO₂) or a mixture of cerium dioxide (CeO₂) and cerium (III) oxide (Ce₂O₃); more preferably cerium dioxide (CeO₂). However, with regard to the selective hydrogenation catalyst of the present invention, "selective hydrogenation" refers to a reaction where only an oxygen-containing polar functional group(s) of a compound is selectively hydrogenated, provided that the compound has both a hydrogenation reaction site(s) such as a carbon-carbon double bond and an oxygen-containing polar functional group(s) such as an epoxy bond.

As for the silver component-cerium oxide complex "AgNPs@CeO₂," no particular limitation is imposed on a manner by which the cerium oxide is supported on the surfaces of the silver component particles if the cerium oxide is supported on the surfaces of the silver component particles. In fact, various configurations are possible. For example, the cerium oxide may be dispersedly supported on the surfaces of the silver component particles as the cores to form a sea-island structure; the cerium oxide may also cover a significant portion of the surfaces of the cores, but partially allow the silver component particles as the cores to be exposed; or the cerium oxide may even completely cover all the surfaces of the cores. Further, the silver component-cerium oxide complex "AgNPs@CeO₂" may form a cluster of the silver component-cerium oxide particles, and the cerium oxide may further cover the surface of the silver component if the silver component is exposed on the surfaces of the cluster particles.

In accordance with the aforementioned supporting configurations, the cerium oxide as a component of the silver component-cerium oxide complex "AgNPs@CeO₂" may be present in the state of particles; a state where the adjacent particles are joined to one another; a state where the joining of the adjacent particles has escalated in such a manner that the core surfaces are covered by the particles in a net-like fashion, and the silver component particle surfaces are then exposed from such net-like structure; or even a state where the cerium oxide as a layer completely covers the silver component particle surfaces.

The state of the cerium oxide is also affected by a molar ratio of Ag/CeO₂ described later. Nevertheless, it is desired that the cerium oxide particles cover the silver component particles to such an extent that the silver component particles as the cores are capable of acting on a substrate through the cerium oxide covering the surfaces of the silver component particles.

Here, no particular limitation is imposed on the manner by which the cerium oxide covers the cores made of the silver component particles, as long as the silver component particles as the cores are capable of acting on the substrate through the cerium oxide covering the surfaces of the silver component particles. For example, as compared to the state where the cerium oxide completely covers the cores made of the silver component particles, the state where the cores made of the silver component particles are partially exposed may lead to a superior reactivity and selectivity of a an oxygen-containing polar functional group(s) of a compound having a hydrogenation reaction site(s) and such oxygen-containing polar functional group(s).

Further, no particular limitation is imposed on the cerium oxide particles covering the cores made of the silver component particles as the component of the silver component-cerium oxide complex "AgNPs@CeO₂." In fact, the cerium oxide particles may be primary or secondary particles. It is preferred that the average particle diameter of the cerium oxide particles be 2 to 40 nm, more preferably 5 to 15 nm. Here, the term "average particle diameter" in the present application refers to an average value of the diameters of an arbitrary number of the particles that is observed through electron micrograph observation. An average cerium oxide particle diameter (shell particle diameter) of not smaller than 2 nm is effective in improving the selectivity, and an average cerium oxide particle diameter of not larger than 40 nm is effective in improving the activity.

Here, no particular limitation is imposed on the silver component particles, as long as the particles contain silver (Ag). In fact, the silver component particles may be those made of metal silver; those in an oxide state; or those in a complex state where a part of metal silver is in the oxide state. That is, the silver component is selected from metal silver, silver oxides and a combination thereof.

The silver component particles may be primary particles or secondary particles. It is preferred that the average particle diameter of the silver component particles be 5 to 30 nm, more preferably 7 to 20 nm.

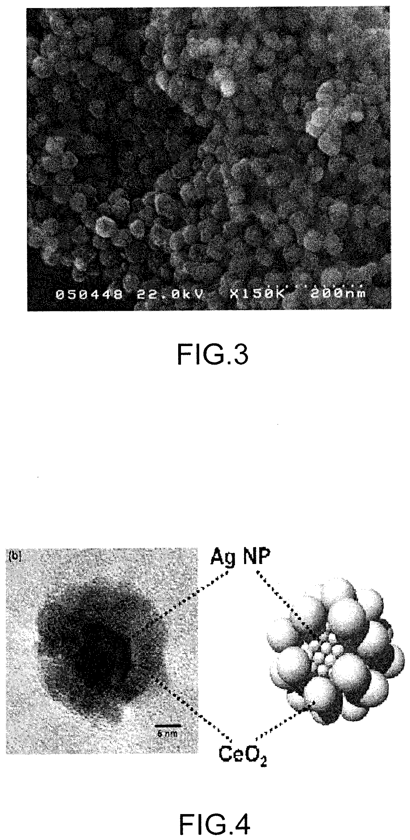

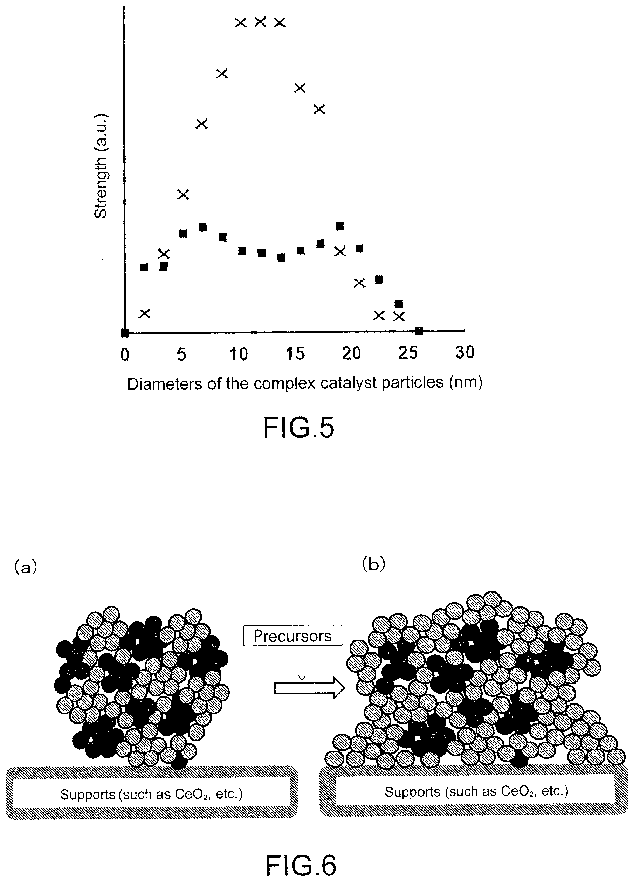

FIG.3 to FIG.5 show a specific structure of the silver component-cerium oxide complex "AgNPs@CeO₂." FIG.3 is an FE-SEM image as a photograph for measurement; FIG.4 is a TEM image as a photograph for measurement. Further, FIG.5 is an analysis chart showing a composition distribution of the components by STEM-EDS. As shown in FIG.3 to FIG.5, it became clear that the specific structure of the silver component-cerium oxide complex "AgNPs@CeO₂" supported on the metal oxide support was a near-spherical core-shell type structure in which the silver component existed as the core, whereas the cerium oxide component existed as a shell. Here, in FIG.4, "Ag NP" represents silver component particles.

### ([Ag/CeO₂] molar ratio of silver component-cerium oxide complex)

As for a composition ratio between silver and cerium oxide among the silver component particles of the silver component-cerium oxide complex "AgNPs@CeO₂," it is preferred that a molar ratio [Ag/CeO₂] be 0.25 to 3.0, more preferably 0.5 to 2.0, and yet more preferably 0.75 to 1.0, the molar ratio being obtained in terms of the number of moles of silver (Ag) as a metal/the number of moles of the cerium oxide (CeO₂). Preferably, when the [Ag/CeO₂] molar ratio is not lower than 0.25, it is effective in improving the conversion rate of the oxygen-containing polar functional group(s) of the compound having the hydrogenation reaction site(s) and such oxygen-containing polar functional group(s), whereas when the [Ag/CeO₂] molar ratio is not higher than 3.0, it is then effective in improving the selectivity of the oxygen-containing polar functional group(s) of the compound having the hydrogenation reaction site(s) and such oxygen-containing polar functional group(s).

### (Particle diameter of silver component-cerium oxide complex "AgNPs@CeO₂")

When the silver component-cerium oxide complex "AgNPs@CeO₂" is formed into the shape of a true sphere, it is desired that the average particle diameter thereof be 5 to 100 nm, more preferably 10 to 60 nm, though no particular limitation is imposed on such average particle diameter. An average particle diameter of not smaller than 5 nm is effective in improving the selectivity of the oxygen-containing polar functional group(s) (referred to as "selectivity" hereunder) of the compound having the hydrogenation reaction site(s) and such oxygen-containing polar functional group(s), whereas an average particle diameter of not larger than 100 nm is effective in improving the conversion rate of the oxygen-containing polar functional group(s) (referred to as "conversion rate" hereunder) of the compound having the hydrogenation reaction site(s) and such oxygen-containing polar functional group(s).

### (Core-shell type structure of silver component-cerium oxide complex "AgNPs@CeO₂")

No particular limitation is imposed on the structure of the silver component-cerium oxide complex "AgNPs@CeO₂." In fact, the silver component-cerium oxide complex "AgNPs@CeO₂" may appropriately employ various kinds of structures in accordance with the type of substrate for selective hydrogenation reaction. For example, when a so-called "core-shell type structure" is employed as the structure of the silver component-cerium oxide complex "AgNPs@CeO₂," it is preferred that the average particle diameter be 10 to 100 nm, more preferably 20 to 60 nm, though no particular limitation is imposed on the size of such structure. Here, an average particle diameter of not smaller than 10 nm is effective in improving the selectivity of the oxygen-containing polar functional group(s) of the compound having the hydrogenation reaction site(s) and such oxygen-containing polar functional group(s), whereas an average particle diameter of not larger than 100 nm is effective in improving the conversion rate of the oxygen-containing polar functional group(s) of the compound having the hydrogenation reaction site(s) and such oxygen-containing polar functional group(s).

Particularly, the "core-shell type structure" refers to a two-layered structure where a shell layer made of the cerium oxide particles is formed on the surfaces the core particles made of the silver component particles. However, this does not necessarily mean that the shell layer completely covers the cores. For example, in a case where the shell layer is made of the cerium oxide particles, the structure may be in a state where the cerium oxide particles at least partially surround or cover the cores; or in a state where the shell layer has continuous clearances large enough to allow the substrate to access the silver component particles as the cores, and the core particles made of the silver component particles are thus linked to the outer side of the shell layer through the clearances, even if the surfaces of the cores made of the silver component particles cannot be identified with a transmission electron microscope or the like.

Further, the "state where the shell layer has continuous clearances large enough to allow the substrate to access the silver component particles as the cores" refers to a shell layer uniformly covering the cores, but having fine pores large enough to allow the substrate to react while being acted on by both the silver component particles as the cores and the cerium oxide as the shell layer. Further, the silver component-cerium oxide complex "AgNPs@CeO₂" may also be formed into a cluster made of the particles of the silver component-cerium oxide complex. Furthermore, in a case where the silver component is exposed on the surface of the cluster, such surface may also be further covered by the cerium oxide. As for the configuration of such cluster, it can collectively comprise two or more silver component-cerium oxide particle complexes each having the core-shell type structure. Other than that, the cluster can have a configuration where the cerium oxide component covers the surfaces of two or more silver component particles as the cores.

With respect to the silver component-cerium oxide complex "AgNPs@CeO₂," the sizes of the fine pores of the shell layer are measured through, for example, a gas absorption method using a gas such as nitrogen, helium and krypton. Although no particular limitation is imposed on the sizes of such fine pores, a fine pore diameter only needs to be large enough to allow the substrate molecules to pass therethrough. For example, it is of the size large enough to allow a substrate described later in the working examples to pass therethrough, and it is preferred that an average fine pore diameter be about 0.5 to 5.0 nm. An average fine pore diameter smaller than 0.5 nm is not preferable, because there are compounds that cannot pass through the fine pores of the shell layer and thus may result in a low conversion rate if using an aromatic compound as the substrate. Further, an average fine pore diameter larger than 5.0 nm is also not preferable, because the substrate cannot be sufficiently acted on by both the silver component particles as the cores and the cerium oxide composing the shell layer such that the selectivity of the oxygen-containing polar functional group(s) of the compound having the hydrogenation reaction site(s) and such oxygen-containing polar functional group(s) may be low.

Further, as for the particles of the silver component-cerium oxide complex having the aforementioned core-shell type structure, it is preferred that the average particle diameter of the silver component particles as the cores be 5 to 30 nm, more preferably 7 to 20 nm. When the average particle diameter of the silver component particles is not smaller than 5 nm, it is effective in improving the selectivity of the oxygen-containing polar functional group(s) of the compound having the hydrogenation reaction site(s) and such oxygen-containing polar functional group(s). Meanwhile, when the average particle diameter of the silver component particles is not larger than 30 nm, it is effective in improving the conversion rate of the oxygen-containing polar functional group(s) of the compound having the hydrogenation reaction site(s) and such oxygen-containing polar functional group(s). In addition, as for the cerium oxide particles composing the shell layer, it is preferred that such cerium oxide particles surround and cover the silver component particles as the cores, in the form of a shell layer having a thickness of 2 to 40 nm, more preferably 5 to 15 nm. A shell layer thickness of not smaller than 2 nm is effective in improving the selectivity of the oxygen-containing polar functional group(s) of the compound having the hydrogenation reaction site(s) and such oxygen-containing polar functional group(s), whereas a shell layer thickness of not larger than 40 nm is effective in improving the conversion rate of the oxygen-containing polar functional group(s) of the compound having the hydrogenation reaction site(s) and such oxygen-containing polar functional group(s).

When the silver component-cerium oxide complex "AgNPs@CeO₂" of the present invention employs the so-called core-shell type structure, the silver component particles as the cores may be completely covered by the cerium oxide particles or partially exposed, as described above.

Further, in a case where the silver component-cerium oxide complex "AgNPs@CeO₂" employs the so-called core-shell type structure; and the cerium oxide composing the shell layer is in the form of particles, it is preferred that the average particle diameter of the cerium oxide be 2 to 40 nm, more preferably 5 to 15 nm. When the average particle diameter of the cerium oxide particles (shell average particle diameter) is not smaller than 2 nm, it is effective in improving the selectivity of the oxygen-containing polar functional group(s) of the compound having the hydrogenation reaction site(s) and such oxygen-containing polar functional group(s). Meanwhile, when the average particle diameter of the cerium oxide particles is not larger than 40 nm, it is effective in improving the conversion rate of the oxygen-containing polar functional group(s) of the compound having the hydrogenation reaction site(s) and such oxygen-containing polar functional group(s).

In a case where the silver component-cerium oxide complex "AgNPs@CeO₂" has the core-shell type structure, although no particular limitation is imposed on the shape thereof, the structure may be approximately formed into the shape of a true sphere. In this way, when the particle of the silver component-cerium oxide complex "AgNPs@CeO₂" is approximately formed into the shape of a true sphere, there are expected the following functions and effects.

That is, as for the silver component-cerium oxide complex "AgNPs@CeO₂" used in the selective hydrogenation catalyst of the present invention, the high selectivity thereof to the oxygen atom of the oxygen-containing polar functional group(s) is possibly derived from the fact that while hydrogen species with high polarities are efficiently generated through heterolytic cleavage of hydrogen molecule, hydrogen species with low polarities are barely generated through homolytic cleavage of hydrogen molecule. Heterolytic cleavage of hydrogen molecule takes place in the vicinity of the boundary surface between the silver component particles and the cerium oxide, while homolytic cleavage of hydrogen molecule takes place on the surface of the silver component particles.

When the silver component-cerium oxide complex "AgNPs@CeO₂" used in the present invention is a core-shell-type silver component-cerium oxide complex "AgNPs@CeO₂" where the silver component particle surfaces of the complex are appropriately covered by the cerium oxide, it is considered that most active sites enabling the access by the substrate as the reacting molecules are located in the vicinity of the boundary surface between the silver component particles and the cerium oxide, and that there are few sole silver component particle surfaces causing homolytic cleavage of hydrogen molecule.

### (Shape of silver-cerium oxide complex "AgNPs@CeO₂")

When the silver-cerium oxide complex "AgNPs@CeO₂" used in the present invention is approximately formed into the shape of a true sphere, the cerium oxide can easily cover the silver component particles in a uniform manner such that there can be easily formed a catalyst with which the aforementioned functions can be easily obtained, and that there may be obtained a catalyst with a high selectivity.

However, even when the silver-cerium oxide complex "AgNPs@CeO₂" used in the present invention is approximately formed into the shape of a true sphere and has the core-shell structure, the cerium oxide may cover the silver component particles in a complete manner, or in an incomplete manner such that clearances exist between the adjacent cerium oxide particles. When the silver component particles are partially exposed, while no particular limitation is imposed on the state of their exposures, a slight exposure may lead to a superior reactivity and selectivity with respect to the oxygen-containing polar functional group(s) of the compound having the hydrogenation reaction site(s) and such oxygen-containing polar functional group(s).

### (Metal oxide support)

Although no particular limitation is imposed on the metal oxide support as a support (base material) of the selective hydrogenation catalyst of the present invention, the metal oxide support may be an oxide of cerium, zirconium, titanium, aluminum, silicon, tungsten or iron; or a composite oxide made of two or more kinds of these metals. Here, with regard to a later-described selective hydrogenation catalyst "AgNPs@CeO₂-D" produced through the reverse micelle technique as an example of a production method of such selective hydrogenation catalyst "AgNPs@CeO₂-D" described later, it is preferred that a cerium-containing oxide be used as the metal oxide support.

When a cerium-containing oxide is used as the metal oxide support, there can be obtained the selective hydrogenation catalyst "AgNPs@CeO₂-D" simply through a drying and calcining steps without using, for example, a binder component or a surfactant. Here, cerium dioxide (CeO₂) was used as the metal oxide support in the later-described working examples, and the inventors of the present invention have found that the silver component-cerium oxide complex "AgNPs@CeO₂" can be easily supported on cerium dioxide (CeO₂). Although no particular limitation is imposed on such cerium-containing oxide as the metal oxide support, there can be used, for example, a cerium-zirconium composite oxide, a cerium-titanium composite oxide, a cerium-aluminum composite oxide and a cerium-silicon composite oxide, other than a pure cerium oxide. It is particularly preferred that cerium dioxide (CeO₂) be used as the metal oxide support.

When using a cerium-containing composite oxide as the metal oxide support, a metal oxide(s) other than the cerium dioxide (CeO₂) and the silver component-cerium oxide complex "AgNPs@CeO₂" will interact with each other in a manner as follows. That is, the inventors of the present invention have found that cerium dioxide (CeO₂) as the metal oxide support differs from a metal oxide support other than cerium dioxide (CeO₂) in ease of supporting the silver component-cerium oxide complex "AgNPs@CeO_{2.}" Further, by utilizing the difference in the capability of supporting the silver component-cerium oxide complex "AgNPs@CeO₂" between the cerium oxide and other metal oxide support, the composition of the cerium oxide versus other metal oxide support(s) can be modified to finely control the dispersibility of the silver component-cerium oxide complex "AgNPs@CeO₂" on the metal oxide support.

Although no particular limitation is imposed on the various properties of the metal oxide support such as the adsorptive property thereof, the adsorptive property of such metal oxide support may be 0.1 to 300 m²/g in terms of BET value; and 0.1 to 100 µm in terms of average particle diameter. Here, in a case where the selective hydrogenation catalyst is produced by a method employing a coprecipitation technique, it is preferred that the adsorptive property of the metal oxide support be 0.5 to 180 m²/g.

As for such selective hydrogenation catalyst (also referred to as AgNPs@CeO₂-D hereunder), no particular limitation is imposed on the manner by which the silver component-cerium oxide complex "AgNPs@CeO₂" may be supported on the metal oxide support. In fact, there can be employed various configurations in accordance with the configuration of the metal oxide support. Further, as for the amount of the silver component-cerium oxide complex "AgNPs@CeO₂" supported on the metal oxide support in the selective hydrogenation catalyst "AgNPs@CeO₂-D," it is preferred that such supported amount be 0.1 to 10 wt% in terms of the amount of the silver component through metal conversion.

It is obvious that the selective hydrogenation catalyst "AgNPs@CeO₂-D" of the present invention has a particle diameter larger than that of the silver component-cerium oxide complex "AgNPs@CeO₂," and can thus be easily separated after being used for selective hydrogenation reaction. That is, the catalyst of the present invention also exhibits a favorable reusability.

### <Production method of selective hydrogenation catalyst "AgNPs@CeO₂-D">

While the selective hydrogenation catalyst "AgNPs@CeO₂-D" of the present invention is a catalyst where the silver component-cerium oxide complex "AgNPs@CeO₂" is supported on the metal oxide support, no particular limitation is imposed on the production method thereof. Described hereunder is an example of a method for producing the selective hydrogenation catalyst "AgNPs@CeO₂-D."

As a method for producing the silver component-cerium oxide complex "AgNPs@CeO₂," there can be used not only the known coprecipitation methods that are disclosed in, for example, Journal of Catalysis 282 (2011) 289-298, WO2007/011062 and JP Patent Application 2012-47231 filed by the inventors of the present invention, but also the reverse micelle technique. The silver component-cerium oxide complex "AgNPs@CeO₂" obtained through such production method of the silver component-cerium oxide complex "AgNPs@CeO₂," is then to be supported on the metal oxide support through various kinds of methods, thus obtaining the selective hydrogenation catalyst "AgNPs@CeO₂-D."

Following are examples of the method by which the silver component-cerium oxide complex "AgNPs@CeO₂" may be supported on the metal oxide support. For example, there may be used a method where the silver component-cerium oxide complex "AgNPs@CeO₂" is to be supported on the metal oxide support; or a method where a precursor of the silver component-cerium oxide complex "AgNPs@CeO₂" that has not yet been calcined is to be supported on the metal oxide support, followed by drying and calcining the same.

Further, a known binder component may be used to support the silver component-cerium oxide complex "AgNPs@CeO₂" on the metal oxide support. Particularly, mixing, drying and calcining can be performed after the surfaces of the metal oxide support and the silver component-cerium oxide complex "AgNPs@CeO₂" have been covered by a known method of promoting the binding of the particles.

As for a production method of such selective hydrogenation catalyst "AgNPs@CeO₂-D," preferred is a method where a precursor of the silver component-cerium oxide complex "AgNPs@CeO₂" is to be supported on the metal oxide support, followed by drying and calcining the same. According to this production method, the silver component-cerium oxide complex "AgNPs@CeO₂" can be relatively solidly supported on the metal oxide support without having to use a binder component. However, while the catalyst production method of the present invention is capable of realizing a relatively solid support without using a binder, it does not inhibit the usage of an accessory component such as a binder for supporting the silver component-cerium oxide complex "AgNPs@CeO₂" when carrying out the present invention.

### <Selective hydrogenation catalyst production method using reverse micelle technique >

As an example of the "AgNPs@CeO₂-D" production method using a precursor of the silver component-cerium oxide complex "AgNPs@CeO₂," described hereunder is a production method using the reverse micelle technique. According to such production method of the selective hydrogenation catalyst "AgNPs@CeO₂-D" using the reverse micelle technique, an easy control can be favorably performed on the shape of the silver component-cerium oxide complex "AgNPs@CeO₂" in the selective hydrogenation catalyst "AgNPs@CeO₂-D" where the surfaces of the silver component particles as the cores are appropriately covered by the cerium oxide.

The production method of the selective hydrogenation catalyst using the reverse micelle technique comprises a step for preparing a mixture by adding the metal oxide support to a liquid mixture of a reverse micelle containing the silver component particles and a reverse micelle containing the cerium oxide; a step for drying the mixture thus produced and then forming a catalyst precursor where the silver component-cerium oxide complex is supported on the metal oxide support; and a step of calcining the catalyst precursor thus formed. Each step is described in detail hereunder.

### (Step for producing mixture by adding metal oxide support to reverse micelle mixture)

A liquid mixture of a reverse micelle containing the silver component particles and a reverse micelle containing the cerium oxide is used in the production method of the selective hydrogenation catalyst employing the reverse micelle technique. Here, reverse micelle refers to a state where the surfactant molecules in the liquid phase associate with one another in a granular manner with the hydrophilic groups thereof being located on the inner side and the hydrophobic groups thereof being located on the outer side. When preparing a precursor of the silver component-cerium oxide complex "AgNPs@CeO₂," a reverse micelle of the silver component and a reverse micelle of the cerium component are prepared respectively, followed by producing a mixture using the reverse micelle solution of silver and the reverse micelle solution of cerium. The mixture thus produced is then dried to obtain the precursor of "AgNPs@CeO₂." By further calcining the dried substance thus obtained, there can be obtained the silver component-cerium oxide complex "AgNPs@CeO₂."

In order to prepare the reverse micelle of the silver component, a surfactant is to be added to the liquid phase, followed by stirring the same so as to form the reverse micelle. Next, an aqueous silver salt solution is added to such reverse micelle solution, followed by further stirring the same such that the reverse micelle solution of the silver component can thus be prepared. Although no particular limitation is imposed on the silver salt component as long as the silver salt component used is soluble in water and does not easily cause hardly-soluble precipitation when mixed with an aqueous cerium salt solution, diammine silver (I) nitrate may, for example, be used as such salt.

The reverse micelle of the cerium component is prepared through a similar method. That is, a surfactant is to be added to the solution phase, followed by stirring the same to form the reverse micelle. Next, an aqueous cerium salt solution is added to such reverse micelle solution, followed by further stirring the same such that the reverse micelle solution of a cerium salt can thus be prepared. Although no particular limitation is imposed on the cerium salt as long as the cerium salt used is soluble in water and does not easily cause hardly-soluble precipitation when mixed with the aqueous silver salt solution, preferred is a trivalent salt, and cerium nitrate (III) may, for example, be used as such salt.

Although no particular limitation is imposed on the liquid composing the liquid phase used in such reverse micelle technique, it is preferred that such liquid be an organic solvent. As such organic solvent, a non-polar organic solvent such as cyclohexane, hexane or dodecane is preferred. Further, as the surfactant, it is preferred that a non-ionic surfactant such as poly (oxyethylene) nonylphenylether be used.

### (Step for forming catalyst precursor)

The precursor of the silver component-cerium oxide complex "AgNPs@CeO₂" that is obtained in the aforementioned step is again dispersed in a liquid phase, followed by mixing the dispersion liquid thus obtained with the metal oxide support, and then drying the same, thus forming the catalyst precursor.

In the step for forming the catalyst precursor, no particular limitation is imposed on the solvent as the liquid phase and a drying condition(s). For example, as the solvent, there can be used ethanol, tetrahydrofuran or the like. Further, an atmosphere as one of the drying conditions can be that of the atmospheric air, a vacuum atmosphere, a nitrogen atmosphere or the like. Here, the temperature also as a drying condition can be that of a room temperature, and drying can be performed through natural drying at such room temperature or using a drying device such as a drum type dryer, a decompression drying apparatus or a spray dryer.

### (Step of calcining catalyst precursor)

The production method of the selective hydrogenation catalyst using the reverse micelle technique comprises a step for calcining the catalyst precursor obtained in the aforementioned step. In such calcining step, the temperature as a calcining condition is within a range of 300 to 600°C; and the atmosphere for calcining may be that of the atmospheric air or, for example, an inert gas atmosphere such as a nitrogen atmosphere. In addition, after obtaining the mixture by mixing the reverse micelle solution of silver and the reverse micelle solution of cerium, the silver component-cerium oxide complex "AgNPs@CeO₂" in the liquid mixture of the silver component-cerium oxide complex "AgNPs@CeO₂" may be further subjected to a drying step for drying the mixture thus obtained. Performing such drying step is preferable, since the silver component-cerium oxide complex "AgNPs@CeO₂" may be further stabilized.

Here, the catalyst precursor may be that with the silver component-cerium oxide complex "AgNPs@CeO₂" being supported on the metal oxide support; or that obtained by calcining the silver component-cerium oxide complex "AgNPs@CeO₂," and then having the same supported on the metal oxide support using a binder component or the like if necessary. In such case, there can be established a further stabilized state by which the silver component-cerium oxide complex "AgNPs@CeO₂" is supported on the metal oxide support, which composes the selective hydrogenation catalyst "AgNPs@CeO₂-D" of the present invention.

However, as for the aforementioned production method of the selective hydrogenation catalyst using the reverse micelle technique, there exists a disadvantage that fulminating silver will be generated during the production steps. That is, the "fulminating silver" generated in the production steps of the production method of the selective hydrogenation catalyst using the reverse technique, refers to a mixture of silver nitride (Ag₃N) and silver amide (AgNH₂), and is explosive. Such "fulminating silver" is significantly sensitive to an external impact and will cause an enormous explosion even when there only occurs a slight friction. Therefore, the production method of the selective hydrogenation catalyst using the reverse micelle technique may lack safety, and thus needs to be further improved in terms of mass productivity.

The present invention has been made to address such technical issue. That is, the present invention provides an excellently safe production method of the selective hydrogenation catalyst where no fulminating silver is generated and only simple and easy processes are required.

### <Production method of selective hydrogenation catalyst using coprecipitation technique>

The production method of the present invention for producing the selective hydrogenation catalyst using the coprecipitation technique, comprises: a (i) step for preparing a raw material solution containing a mixed salt of the silver salt and the cerium salt; a (ii) step for forming on the metal oxide support coprecipitation mixed particles of a silver compound and a cerium compound, by mixing the aforementioned raw material solution and the metal oxide support under the presence of a coprecipitation agent acting on both the silver and cerium ions; a (iii) step for forming the catalyst precursor by further adding the cerium salt to the coprecipitation mixed particles on the metal oxide support under the presence of the coprecipitation agent, and then allowing the precipitate of the cerium compound to be supported on the coprecipitation mixed particles; and a (iv) step for calcining the catalyst precursor. Each of these steps is described in detail hereunder.

### (Step (i) preparing raw material solution containing mixed salt of silver salt and cerium salt)

The production method of the present invention for producing the selective hydrogenation catalyst using the coprecipitation technique comprises the step (i). The step (i) is to prepare the raw material solution containing the mixed salt of the silver salt and the cerium salt. In the step (i), the silver salt and the cerium salt that are used contain silver ions and cerium ions respectively, and are both electrolyte salts generated when combined with negative ions. It is preferred that the negative ions contained in the silver salt and the cerium salt be common negative ions. Particularly, a salt containing the silver salt and the cerium salt is defined as the mixed salt. Moreover, a solution obtained by dissolving such mixed salt in a solvent is defined as the raw material solution in the step (i).

It is preferred that the silver salt and the cerium salt that are contained in the mixed salt are salts dissoluble in a solvent, especially in water. No particular limitation is imposed on the silver salt and the cerium salt as long as they contain silver ions and cerium ions respectively. Specifically, examples of the silver salt include silver nitrate, silver sulfate, silver acetate, silver chlorate and silver lactate. Examples of the cerium salt include cerium chloride, cerium nitrate, cerium sulfate, cerium acetate, cerium oxalate and cerium trifluoromethanesulfonate. Even among the examples listed above, silver nitrate is preferably used as the silver salt, and cerium nitrate is preferably used as the cerium salt in terms of their solubilities in water and eases in handling the same. Further, there may be used a single species of silver salt and a single species of cerium salt respectively; or there may be used by mixture not less than two kinds of silver salt and not less than two kinds of cerium salt respectively. Further, the silver salt and the cerium salt may also contain electrolyte salt hydrates.

As for the amount of each of the silver salt and the cerium salt that are used, the cerium salt is used in an amount of 1.0 to 10.0 mol, preferably 3.0 to 5.0 mol with respect to 1.0 mol of the silver salt. It is preferred that the cerium salt be used in an amount of not smaller than 1.0 mol with respect to 1.0 mol of the silver salt, because the cerium oxide particles can thus be supported on the surfaces of the silver component particles. Further, it is preferred that the cerium salt be used in an amount of not smaller than 10.0 mol with respect to 1.0 mol of the silver salt, because this will make it easy to form the silver component-cerium oxide complex having the core-shell type structure where the silver component particles serve as the cores; and the shell layer is made of the cerium oxide component.

No particular limitation is imposed on the solvent used to prepare the raw material solution, as long as the solvent is capable of sufficiently dissolving the mixed salt and being used to prepare a homogeneous raw material solution. Examples of such solvent may variously include water and alcohol (single or mixed solvent made of methanol, ethanol, ethylene glycol and the like), among which water is particularly preferable. Here, when preparing the raw material solution, the preparation temperature, stirring speed, pH and the like can be appropriately set in accordance with various conditions such as the molar ratios of the silver salt and cerium salt composing the mixed salt; and the amount of the mixed salt contained in the raw material solution.

### (Step (ii) forming coprecipitation mixed particles of silver compound and cerium compound on metal oxide support)

The production method of the present invention for producing the selective hydrogenation catalyst using the coprecipitation technique comprises the step (ii). The step (ii) is to form on the metal oxide support the coprecipitation mixed particles composed of the silver compound and the cerium compound, by mixing the raw material solution prepared in the step (i) and the metal oxide support under the presence of a coprecipitation agent. The coprecipitation mixed particles can be formed by suspending the metal oxide support in a water solution containing the coprecipitation agent, and then adding to the suspension thus obtained the raw material solution of the step (i) that contains the mixed salt. In this way, precipitated on the metal oxide support from the raw material solution containing the mixed salt, are the "coprecipitation mixed particles" as the mixed particles containing a hardly-soluble silver compound and a hardly-soluble cerium compound.

Particularly, "hardly-soluble" refers to a state where a solubility is lower than those of the silver salt and the cerium salt in the raw material solution. More particularly, "hardly-soluble" is defined as a solubility of not higher than 1.0 g/100 g (H₂O) at 25°C, provided that water is used as the solvent. Here, no particular limitation is imposed on the coprecipitation agent used in the step (ii), as long as such coprecipitation agent is a compound or electrolyte salt capable of providing negative ions with which the precipitation can be generated when acted upon by the metal positive ions of both the silver ions and cerium ions contained in the raw material solution. Specifically, it is preferred that the silver compound and the cerium compound be compounds having the solubility of, for example, not higher than 1.0 g/100 g (H₂O) at 25°C.

Examples of the negative ions capable of generating the precipitation when acted upon by both the silver ions and cerium ions contained in the raw material solution, include hydroxy-ions, carboxylate ions, sulfate ions, acetate ions, halogen ions, carbonate ions and bicarbonate ions. The coprecipitation agent is appropriately selected from, for example, oxalate, tartrate, citrate, sulfate, acetate, lactate, halide, carbonate, bicarbonate, hydroxide and various organic acid salts. Particularly, examples of the compound or electrolyte salt that can be used as the coprecipitation agent, include caustic soda, sodium carbonate, caustic potash, oxalic acid, carbonic acid, potassium carbonate, ammonium carbonate, cesium hydroxide, ammonium hydroxide and ammonium.

While no particular limitation is imposed on the amount of the coprecipitation agent used, such amount is appropriately determined based on the chemical equivalent of the silver oxide and cerium oxide in the raw material solution prepared in the step (i). In view of the chemical equivalent of the silver and cerium, it is preferred that there be first calculated the amount (theoretical value) of the coprecipitation agent capable of yielding the "coprecipitation mixed particles" composed of sufficient silver ion precipitations and cerium ion precipitations, and that an amount with a 10 to 50% increase from the amount (theoretical value) of the coprecipitation agent be then defined as the amount of the coprecipitation agent to be used. Moreover, the coprecipitation agent can be supplied to the raw material solution on the metal oxide support, in the form of a solution, preferably a water solution.

No particular limitation is imposed on the metal oxide support. In fact, the metal oxide support can be an oxide of, for example, cerium, zirconium, titanium, aluminum, silicon, tungsten or iron; or a compound oxide of not less than two of these metals. As for the production method of the present invention for producing the selective hydrogenation catalyst "AgNPs@CeO₂-D" using the coprecipitation technique, it is preferred that a cerium-containing oxide, especially cerium dioxide be used as the metal oxide support.

When using such cerium-containing oxide as the metal oxide support, the coprecipitation mixed particles can be supported on the metal oxide support without having to use, for example, a binder component or a surfactant. Next, by adding the cerium salt solution to the coprecipitation mixed particles, there can be obtained "hardly-soluble cerium compound-supporting coprecipitation mixed particles" with the surfaces of the coprecipitation mixed particles being covered by the cerium salt. However, as for the production method of the present invention for producing the selective hydrogenation catalyst using the coprecipitation technique, the usage of an accessory component such as a binder is not inhibited.

In the working examples described below, CeO₂ was used as the metal oxide support, because the inventors of the present invention had found that CeO₂ could easily support the coprecipitation mixed particles. While no particular limitation is imposed on such cerium-containing oxide as the metal oxide support, there may be used, for example, a cerium-zirconium compound oxide, cerium-titanium compound oxide, cerium-aluminum compound oxide and cerium-silicon compound oxide, other than pure cerium oxide.

Particularly, by using a cerium-containing compound oxide as the metal oxide support, there occurs the following interaction between a metal oxide other than CeO₂ and the coprecipitation mixed particles. That is, the inventors found a difference in ease of supporting the coprecipitation mixed particles between CeO₂ as the metal oxide support and a metal oxide support other than CeO₂, after comparing the same with each other. Further, by utilizing the difference in the capacity of supporting the coprecipitation mixed particles between cerium oxide and other metal oxide supports, the composition of cerium and other metals can be modified to finely control the dispersibility of the coprecipitation mixed particles on the metal oxide support.

While no particular limitation is imposed on the various properties of such metal oxide support such as the adsorptive property thereof, it is preferred that its adsorptive property be 0.5 to 180 [m²/g] in terms of BET value, more preferably 1.0 to 130 [m²/g]. It is preferred that the adsorptive property of the metal oxide support be not lower than 0.5 [m²/g] in terms of BET value, because the coprecipitation mixed particles can be appropriately dispersed on the metal oxide support in such case. Moreover, the amount of the "AgNPs@CeO₂" to be supported on the metal oxide support may also be increased in such case such that the catalytic activity may be improved as well. It is preferred that the adsorptive property of the metal oxide support be not higher than 180 [m²/g], because not only the cerium salt added in the next step can be appropriately dispersed on the coprecipitation mixed particles, but there can also be obtained a catalyst even superior in selectivity.

As for the production method of the selective hydrogenation catalyst using the coprecipitation technique, there can be obtained a selective hydrogenation catalyst "AgNPs@CeO₂-D" capable of exhibiting a superior reactivity and selectivity, by employing a metal oxide support with the adsorptive property of 0.5 to 180 [m²/g] in terms of BET value.

Further, it is preferred that the metal oxide support have an average particle diameter of 0.1 to 100 [µm]. It is preferred that the metal oxide support have an average particle diameter of not smaller than 0.1 [µm], because the coprecipitation mixed particles can be appropriately dispersed on the metal oxide support in such case. Further, it is preferred that the metal oxide support have an average particle diameter of not larger than 100 [µm], because the cerium salt added in the next step can be appropriately dispersed on the coprecipitation mixed particles in such case. Furthermore, when such average particle diameter is not smaller than 0.1 [µm], the selective hydrogenation catalyst can be easily separated when reused; when the average particle diameter is not larger than 100 [µm], it is easy to maintain a favorable dispersion state of such catalyst in the reaction system, and the activity of the catalyst can be improved as well.

### (Step (iii) supporting precipitation of cerium compound on coprecipitation mixed particles)

The production method of the present invention for producing the selective hydrogenation catalyst using the coprecipitation technique comprises the step (iii). The step (iii) is to allow the precipitation of the cerium compound to be supported on the coprecipitation mixed particles formed in the step (ii). In the step (iii), the cerium salt is added, normally in the form of a cerium salt solution, to the "coprecipitation mixed particles" (i.e. hardly-soluble compound mixed particles) composed of the silver compound and the cerium compound that have been precipitated on the metal oxide support. In this way, there can be obtained the "hardly-soluble cerium compound-supporting coprecipitation mixed particles" with the surfaces of the coprecipitation mixed particles being further covered by the hardly-soluble cerium compound. Here, in the step (iii), the "hardly-soluble cerium compound-supporting coprecipitation mixed particles" are defined as the catalyst precursor.

As the cerium salt used in the step (iii), there can be employed the cerium salt presented for use in the raw material solution of the step (i). Cerium nitrate is especially preferred in such case. Water is usually used as the solvent of the cerium salt solution. The amount of the cerium salt added is determined based on the amount of cerium contained in the coprecipitation mixed particles that are obtained in the step (ii). In general, the amount of the cerium salt used in the step (iii) is 0.5 to 2 mol with respect to 1 mol of the cerium salt used in the step (i). However, it is preferred that the cerium salt be used in an amount where a molar ratio of "metallic silver (Ag) / cerium (Ce)" becomes 0.25 to 3.0 in the hardly-soluble cerium compound-supporting coprecipitation mixed particles, provided that such molar ratio is a molar ratio of silver expressed in terms of metallic silver to the cerium salt expressed in terms of cerium (Ce).

In the step (iii), there may be repeated multiple times the step of adding the cerium salt solution to the surfaces of the coprecipitation mixed particles supported on the metal oxide support. By repeating multiple times the step of adding the cerium salt solution, a sufficient amount of the cerium compound can be supported on the surfaces of the coprecipitation mixed particles and on the metal oxide support. By having the hardly-soluble cerium compound supported on the surfaces of the coprecipitation mixed particles, there can be formed coprecipitation mixed particles supporting the hardly-soluble cerium compound having the core-shell type structure where the silver component particles serve as the cores, and the cerium compound particles are dispersed on the entire surfaces of such silver salt particles as the cores.

In the step (iii), hardly-soluble cerium salt-supporting coprecipitation mixed particles are formed as follows. That is, the cerium salt solution is to be added, under the presence of the coprecipitation agent, to the surfaces of the coprecipitation mixed particles supported on the metal oxide support, thereby causing the hardly-soluble cerium salt to be precipitated on the coprecipitation mixed particles, thus obtaining the cerium salt-supporting coprecipitation mixed particles.

FIG.6 is a diagram of a model showing the step of forming the cerium salt-supporting coprecipitation mixed particles. As shown in FIG.6 (a), supported on the metal oxide support are the coprecipitation mixed particles containing the precursor particles of the silver component particles (i.e. hardly-soluble silver compound) and the precursor particles of the cerium dioxide particles (i.e. hardly-soluble cerium compound). As shown in FIG.6 (a), such coprecipitation mixed particles include the mixed particles having the core-shell type structure where the precursors of the silver component particles (i.e. hardly-soluble silver compound) serve as the cores, and the precursor particles of cerium dioxide (i.e. hardly-soluble cerium compound) are dispersed on the entire surfaces of such silver component particles as the cores; and the mixed particles having a structure where the precursors of the silver component particles serve as the cores, and the precursor particles of cerium dioxide are partially dispersed on the surfaces of the precursors of the silver component particles as the cores. The type of the mixed particles employed shall be determined based on the dispersion state of the silver component precursor particles and the cerium dioxide precursor particles, and on the number of the silver component precursor particles and the cerium dioxide precursor particles (mole number). Further, the mixed particles may also be in a cluster-like state where multiple mixed particles having the core-shell type structure have come together.

In the step (iii), by adding the cerium salt to the surfaces of the coprecipitation mixed particles under the presence of the coprecipitation agent, the cerium salt adsorbs onto the precipitated silver compound and cerium compound particles composing the coprecipitation mixed particles. As shown in FIG.6 (b), the cerium salt is precipitated and supported on the silver compound (precursor of silver component) particles as the surfaces of the coprecipitation mixed particles. By having the cerium salt supported on the silver compound particles on the surfaces of the mixed particles, there can be formed the cerium salt-supporting coprecipitation mixed particles having a structure where the silver compound particles serve as the cores, and the cerium compound (precursor of cerium dioxide) particles are supported on the surfaces of such silver compound particles as the cores. Meanwhile, as a result of having the cerium salt supported on the cerium compound particles on the surfaces of the coprecipitation mixed particles, the layer of the cerium compound particles will become even thicker such that the diameter of the coprecipitation mixed particles may reach about 100 to 900 nm.

Further, by adding the cerium salt solution to the surfaces of the coprecipitation mixed particles supported on the metal oxide support, the hardly-soluble cerium salt will enter in between the coprecipitation mixed particles and the metal oxide support. Due to the hardly-soluble cerium salt that has thus entered in between the coprecipitation mixed particles and the metal oxide support, the coprecipitation mixed particles and the metal oxide support can be firmly fixed.

Moreover, in order to generate sufficient silver ion precipitations and cerium ion precipitations in the step (iii), the preparation temperature, stirring speed, pH and the like can be appropriately adjusted in accordance with various conditions such as the molar ratios of the silver compound and cerium compound composing the coprecipitation mixed particles; the amount of the mixed salt contained in the raw material solution; and the type and amount of the coprecipitation agent used.

No particular limitation is imposed on a pH adjuster used to adjust pH as long as there can be generated sufficient silver ion precipitations and the cerium ion precipitations. In fact, such pH adjuster can be appropriately selected in accordance with various conditions such as the amount of the mixed salt contained in the raw material solution; and the type and amount of the coprecipitation agent used. Examples of such pH adjuster include various carbonate, hydrogen carbonate, alkali metal salt and ammonium salt. Although no particular limitation is imposed on the pH value adjusted by the pH adjuster, it is preferred that such pH value be 8.0 to 11.0 in the step of generating the hardly-soluble cerium salt-supporting coprecipitation mixed particles.

Meanwhile, a dispersant may also be used if needed. No particular limitation is imposed on the dispersant used. In fact, the dispersant is appropriately selected from, for example, a water-soluble polymer and various surfactants. Specific examples of such dispersant include water-soluble polymers such as polyvinyl alcohol, polyvinyl pyrrolidone, and polyethylenimine; and non-ionic surfactants such as polyoxyethylene alkyl ether, alkyl mono glyceryl ether, alkyl polyglycoside and fatty acid diethanolamide.

In the step (iii), the hardly-soluble cerium salt-supporting coprecipitation mixed particles obtained are washed if necessary, and then dispersed in the liquid phase again, followed by drying the same such that the catalyst precursor can be formed.

In the step for forming the catalyst precursor, no particular limitation is imposed on the solvent used as the liquid phase; and the drying conditions. Examples of such solvent include ethanol, tetrahydrofuran or the like. Further, an atmosphere as a drying condition can be that of the atmospheric air, a vacuum atmosphere, a nitrogen atmosphere or the like. Here, the temperature also as a drying condition can be that of a room temperature, and drying can be performed through natural drying at such room temperature or using a drying device such as a drum type dryer, a decompression drying apparatus or a spray dryer.

### (Step (iv) calcining catalyst precursor)

In the step for calcining the catalyst precursor obtained in the step (iii), the temperature as a calcining condition is 300 to 600°C, and the atmosphere for calcining such catalyst precursor may be that of the atmospheric air or, for example, an inert gas atmosphere such as a nitrogen atmosphere. In this way, there can be obtained the selective hydrogenation catalyst "AgNPs@CeO₂-D" by only drying and calcining the cerium salt-supporting coprecipitation mixed particles that are supported on the metal oxide support. Here, an accessory component such as a binder is not prohibited from being used at the time of producing the "AgNPs@CeO₂-D."

### <Method of selectively hydrogenating compound having oxygen-containing polar functional group(s)>

Next, described is a method of the present invention for selectively hydrogenating a compound having a hydrogenation reaction site(s) and an oxygen-containing polar functional group(s). Such selective hydrogenation method comprises a step of bringing the compound having the hydrogenation reaction site(s) and the oxygen-containing polar functional group(s) into contact with a hydrogen gas in the liquid phase under the presence of the selective hydrogenation catalyst "AgNPs@CeO₂-D". The step is described hereunder.

### (Substrate: compound having oxygen-containing polar functional group(s))

In the method for selectively hydrogenating the compound having the oxygen-containing polar functional group(s), no particular limitation is imposed on the compound as the substrate having the hydrogenation reaction site(s) and the oxygen-containing polar functional group(s) that are selectively hydrogenated, as long as the compound has both the hydrogenation reaction site(s) and the oxygen-containing polar functional group(s). Here, the compound having the hydrogenation reaction site(s) and the oxygen-containing polar functional group(s) refers to a type of compound having a hydrogenation reaction site(s) such as a carbon-carbon double bond and an oxygen-containing polar functional group(s) such as an epoxy bond, and only allowing its oxygen-containing polar functional group(s) to be selectively hydrogenated.

Specifically, the compound as the substrate having the hydrogenation reaction site(s) and the oxygen-containing polar functional group(s) that are selectively hydrogenated, contains the hydrogenation reaction site(s) such as the carbon-carbon double bond, and may even contain an aromatic ring. The oxygen-containing polar functional group(s) may also be contained in the aforementioned compound as a substituent bonded to aromatic series. Examples of the hydrogenation reaction site(s) include the carbon-carbon double bond. Examples of a group having the carbon-carbon double bond include an alkenyl group with 2 to 6 carbon atoms. Further, such carbon-carbon double bond may be that found in a chainlike group, or that found in a carbon ring or a hetero ring. Such carbon or hetero ring may be that with two or more aromatic rings e.g. benzene rings being condensed. Particularly, there may be used styrene, cis-stilbene, trans-stilbene, divinylbenzene, vinylnaphthalene, vinylbiphenyl, dihydronaphthalene, indole, skatole, benzimidazole, quinoline and benzothiophene. Especially, superior effects have been confirmed in compounds obtained by inserting the following oxygen-containing polar functional group(s) into the compounds such as styrene, trans-stilbene and indole having the carbon-carbon double bonds.

Examples of such oxygen-containing polar functional group(s) include aldehyde group, nitro group, epoxy group and carbonyl group. Here, nitro group and carbonyl group may be those formed together with carbon or nitrogen contained in carbon or hetero ring.

As a compound having nitro group as the oxygen-containing polar functional group(s), effects have been confirmed in aromatic compounds having the carbon-carbon double bonds and nitro groups. Particularly, the significant effects of the present invention have been confirmed in compounds having nitrostyryl groups.

As a compound having aldehyde group as the oxygen-containing polar functional group(s), effects have been confirmed in aromatic compounds having the carbon-carbon double bonds and aldehyde groups. Examples of such compound include 3-vinylbenzaldehyde, cinnamaldehyde, geranial, citral, neral, perillylaldehyde, crotonaldehyde, trans-2-heptenal, methacrolein, trans-2-methyl-2-butenal, 3-methyl-2-butenal, 1-cyclohexenecarboaldehyde, 3-(4-methoxyphenyl) propenal, 3-(4-chlorophyll)propenal and 3-cyclohexenecarboaldehyde.

Further, as a compound having epoxy group as the oxygen-containing polar functional group(s), effects have been confirmed in compounds having the carbon-carbon double bonds and epoxy groups. Examples of such compound include trans-stilbeneoxide, CIS-stilbeneoxide, styrene oxide, trans-β-methylstyrene oxide, 4-methylstyrene oxide, 4-fluorostyrene oxide, 4-chlorostyrene oxide and epoxy-cinnamic-acid-ethyl. Furthermore, as a compound having carbonyl group as the oxygen-containing polar functional group(s), effects have been confirmed in compounds having the carbon-carbon double bonds and carbonyl groups. Examples of such compound include vinyl acetophenone, propenyl acetophenone, butenyl acetophenone and vinyl cyclohexane.

### (Under the presence of selective hydrogenation catalyst in liquid phase)

The selective hydrogenation method of the present invention is performed in a liquid phase. While no particular limitation is imposed on the solvent used, there may also be contained an organic solvent. There may also be used a mixed solvent containing water and such organic solvent. However, an excessive amount of water may cause the liquid phase to be separated into two layers such that the reaction may not proceed easily as the catalyst and the reactive components are now present in different phases. That is, when employing a liquid phase using a mixed solvent, it is preferred that the amount of water be appropriately adjusted, and it is particularly preferred that only an organic solvent be used.

Although no particular limitation is imposed on the organic solvent used in the liquid phase, it is preferred that there be used at least one organic solvent selected from an aliphatic hydrocarbon having 5 to 20 carbon atoms and an aromatic hydrocarbon having 7 to 20 carbon atoms, more preferably 7 to 9 carbon atoms. Examples of such organic solvent include dodecane, cyclohexane, toluene and xylene. Dodecane is especially preferred, because it exhibits a high stability as a solvent for hydrogenation. Further, there may also be used, for example, tetrahydrofuran (THF) known to be freely mixed with water and dissolve many organic compounds and polymers.

### (Step of bringing said compound into contact with hydrogen gas)

According to the selective hydrogenation method of the present invention, said compound as the substrate having the hydrogenation reaction site(s) such as the carbon-carbon double bond and the oxygen-containing polar functional group(s) such as the epoxy bond is brought into contact with a hydrogen gas in the liquid phase. Specifically, other than having the liquid phase aerated with the hydrogen gas, there may also be employed a method of, for example, having both the liquid phase and hydrogen gas received in a reaction container and then pressurizing the hydrogen gas inside such reaction container. Following are examples of the reaction temperature and reaction pressure at the time of bringing the compound having the hydrogenation reaction site(s) and the oxygen-containing polar functional group(s) into contact with the hydrogen gas.

### (Reaction temperature)

When using the selective hydrogenation catalyst of the present invention to selectively hydrogenate the oxygen-containing polar functional group(s) in the compound having the hydrogenation reaction site(s) such as the carbon-carbon double bond and the oxygen-containing polar functional group(s) such as the epoxy bond, no particular limitation is imposed on the reaction temperature as long as the reaction temperature is within a range of common sense. However, it is preferred that such reaction temperature be 20 to 200°C, more preferably 100 to 120°C. This is preferable, because a reaction temperature not lower than 20°C allows the selective hydrogenation reaction to proceed in a favorable manner, and a reaction temperature not higher than 200°C makes it difficult for the aromatic ring(s) of an aromatic compound to be hydrogenated.

### (Reaction pressure)

Further, according to the aforementioned selective hydrogenation method, no particular limitation is imposed on the hydrogen pressure in the hydrogenation reaction system, as long as the hydrogen pressure is within a range of common sense. However, it is preferred that such hydrogen pressure be 0.1 to 3.0 MPa, more preferably 0.5 to 1.0 MPa. This is preferable, because a hydrogen pressure not lower than 0.1 MPa allows the hydrogenation reaction to proceed in a favorable manner, and a hydrogen pressure not higher than 3.0 MPa makes it difficult for the aromatic ring(s) of an aromatic compound to be hydrogenated.

### (Addition of amines)

According to the aforementioned selective hydrogenation method, by further adding amines, side products can be restricted from being generated such that selectively hydrogenated is the oxygen-containing polar functional group(s) in the compound having a hydrogenation reaction site(s) such as a carbon-carbon double bond and an oxygen-containing polar functional group(s) such as an epoxy bond, and both the conversion rate and selectivity thereof can thus be improved.

It is assumed that such performance improvement due to the addition of amines is attributed to the donor effect where amines' unshared electron pair acts on the active sites of the selective hydrogenation catalyst. Preferred as such amines is an alkylamine having 2 to 8 carbon atoms in an alkyl group(s) for substituting the hydrogen atoms. It is assumed that since such alkylamine has a relatively crowded structure, the donor effect will come into play such that the reactivity will be improved. Further, as amines, preferred are secondary amines and tertiary amines, of which tertiary amines are particularly preferred.

Secondary amines as amines used in the selective hydrogenation method include dibenzylamine, dioctylamine or the like; whereas tertiary amines as amines used in the selective hydrogenation method include triethylamine, tributylamine, trihexylamine, trioctylamine or the like. It has been confirmed that triethylamine among these amines exhibits a superior effect.

Although no particular limitation is imposed on the amount of the amines used in the selective hydrogenation method, it is preferred that the amines be used in an amount of 400 to 1,000 mol% with respect to the compound as the substrate having the oxygen-containing polar functional group(s). An amount greater than 1,000 mol% is not preferable, because the conversion rate may decrease in such case; whereas an amount less than 400 mol% is not preferable either, because the selectivity may not be improved.

### (Addition of inorganic base)

According to the selective hydrogenation method using the selective hydrogenation catalyst of the present invention, it is preferred that an inorganic base be added. It was confirmed that not only the side products could be restricted from being generated, but both the conversion rate and selectivity could also be improved by using an inorganic base(s). It is assumed that such performance improvement due to the addition of an inorganic base(s) is attributed to the fact that the effect of promoting the polar heterolytic cleavage of hydrogen molecule is improved. Examples of such inorganic base include cesium carbonate (Cs₂CO₃), potassium carbonate (K₂CO₃), sodium carbonate (Na₂CO₃) and rubidium carbonate (Rb₂CO₃). Cesium carbonate is preferred because the selectivity and conversion rate will be improved the most. Such inorganic base(s) may be used either independently from or together with the aforementioned amines.

### (Selective hydrogenation of oxygen atom of oxygen-containing polar functional group)

According to the aforementioned selective hydrogenation method, molecular hydrogen generated due to the heterogeneous reaction acts on the oxygen-containing polar functional group(s) at the interface between the cerium oxide and the core composed of the silver component particles in the silver component-cerium oxide complex "AgNPs@CeO₂" that serves as the selective hydrogenation catalyst and is supported on the metal oxide support. That is, in this step where hydrogenation takes place, hydrogenation reaction proceeds as the molecular hydrogen generated due to the heterogeneous reaction selectively reacts to the oxygen-containing polar functional group(s) in the compound having a hydrogenation reaction site(s) such as a carbon-carbon double bond and an oxygen-containing polar functional group(s) such as an epoxy bond. According to the aforementioned selective hydrogenation method, there can be produced a hydrogenated compound in which the oxygen atoms in the compound having a hydrogenation reaction site(s) and an oxygen-containing polar functional group(s) have turned into hydrogen atoms.

### EXAMPLES

While catalysts used in the selective hydrogenation method of the present invention and embodiments of the present invention will be described in more detail herein below, it should be understood that the present inventions are not limited by the embodiments described below and various modifications can be made without departing from the spirit or scope of the present invention.

### [Production example 1] (Production of selective hydrogenation catalyst (I): [AgNPs@CeO₂-D] of the example using reverse micelle technique)

8 ml of poly (oxyethylene) nonylphenylether was added to 20 ml of cyclohexane, followed by adding 2 ml of diamine silver (I) nitrate solution (0.15 mol/L) thereto while agitating the same with a Teflon stirrer. Through further agitation in a more vigorous manner, a reverse micelle solution was thus prepared.

8 ml of poly (oxyethylene) nonylphenylether was added into another container containing 20 ml of cyclohexane while being agitated by a Teflon stirrer, and then 2 ml of cerium nitrate (III) water solution (0.10 mol/L) was added thereto while vigorously agitating the same to prepare another reverse micelle solution. The above described two reverse micelle solutions were mixed under an Ar gas stream, and the mixture thereof was then cooled to room temperature while being agitated for one hour at 60°C. Next, 40ml of ethanol was added thereto, and agitation was further performed for 30 minutes. As the result, a black precipitate was produced.

The black precipitate produced in this manner was separated therefrom through centrifugalization, washed with ethanol, and then dried in vacuum at room temperature to obtain a precursor of AgNPs@CeO₂. [molar number in terms of metal silver/molar number in terms of cerium oxide] of the precursor was observed using Inductively Coupled Plasma (ICP) emission spectroscopy analysis. The result was that Ag/CeO₂=1.1.

0.05g of AgNPs@CeO₂ precursor was taken therefrom and ultrasonically dispersed into 100 ml of ethanol to which 2.0 g of CeO₂ [having BET value: 81.4m²/g, crystallite diameter: 11.0nm, average fine pore diameter: 11.6nm, fine pore volume: 0.24 mL/g, median diameter (d50): 4.37 µm] was added and then agitated for 1 hour at room temperature. [AgNPs@CeO₂-D] precursor was taken from the agitated solution using a centrifugal separator.

The precursor of the selective hydrogenation catalyst "AgNPs@CeO₂-D" was dried under a reduced pressure and then calcined for four hours at 400°C in the ambient atmosphere, thereby obtaining a selective hydrogenation catalyst [AgNPs@CeO₂-D]. The selective hydrogenation catalyst [AgNPs@CeO₂-D] thus obtained was analyzed and found to contain 1.0 wt % of silver out of the selective hydrogenation catalyst [AgNPs@CeO₂-D] through metal conversion.

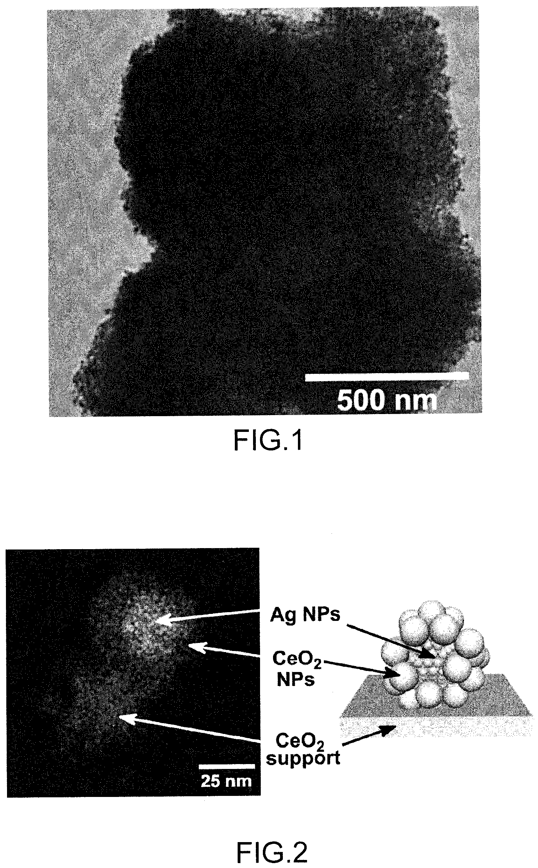

The selective hydrogenation catalyst [AgNPs@CeO₂-D] thus obtained was microstructurally analyzed using STEM and HAADF-STEM. The STEM image and HAADF-STEM image are respectively shown in FIG. 1 and FIG. 2.

As shown in the STEM image of FIG. 1, it is clear that the silver component-cerium oxide complex ┌AgNPs@CeO₂┘ particles are supported on the cerium dioxide (CeO₂) as a metal oxide support. Also, as shown in the HAADF-STEM image of FIG. 2, it is clear that the ┌AgNPs@CeO₂┘ particles supported on the metal oxide support has a core-shell type structure in which the silver component particles serve as the cores, whereas cerium dioxide (CeO₂) serves as a shell. For that reason, with regard to AgNPs@CeO₂-D, cerium component concentration is high on the surfaces of the AgNPs@CeO2 particles, whereas silver component concentration is high at central parts of the particles, as can be seen as well from a later described FIG. 5 showing an analysis chart of STEM-EDS.

### [Production example 2] (Production of comparative selective hydrogenation catalyst (i): silver component-cerium oxide complex ┌AgNPs@CeO₂┘ obtained through reverse micelle technique)

The precursor of AgNPs@CeO₂ obtained through production example 1 was calcined for 4 hours at 400°C in the ambient atmosphere, thereby obtaining 0.06g of AgNPs@CeO₂ used in a comparative example of the present invention.

The silver component-cerium oxide complex obtained in this manner was observed using FE-SEM, and it was found that they had been formed into subglobal shaped particles having a particle diameter of 20 through 40 nm. FIG. 3 shows the image thereof.

Further, the particles of the silver component-cerium oxide complex were observed through TEM. And, it was found that the core composed of the silver component particles existed in the center, and that a shell layer surrounded such core. A TEM image and a schematic view are shown in FIG. 4. Note that the scale of TEM image is 5 nm and that AgNP in the figure indicate silver nanoparticles.

Composition distribution was analyzed using STEM-EDS with respect to each component of the silver component-cerium oxide complex. The analysis indicated a core-shell type structure in which the silver component particles existed as the core, whereas the cerium dioxide (CeO₂) existed as a shell. An analysis chart obtained through STEM-EDS is shown in FIG. 5. Average particle diameter of the silver component particles as the core was found to be around 10 nm. In FIG. 5 as an analysis chart obtained through STEM-EDS, horizontal axis indicates diameters of the silver component-cerium oxide complex particles where the symbol "×" shows a strength of the silver component and the symbol "■" shows a strength of the cerium oxide.

The catalyst obtained in this manner was analyzed using ICP-AES (Inductively Coupled Plasma Atomic Emission Spectroscopy) analysis. Molar ratio of the silver component-cerium oxide complex; that is, [molar number in terms of metal silver/molar number in terms of cerium oxide] was found to be Ag/CeO₂=1.1.

### [Example 1, Comparative example 1]: Selective Hydrogenation Reactions with respect to Aldehyde Groups.

Selective hydrogenation reaction was performed, using a catalyst of the example and that of a comparative example, both of which had been respectively obtained in the production example 1 and production example 2, with respect to citral (2:1 mixture of geranial and neral which are cis-trans isomers with each other) as a substrate containing aldehyde group. And then, the selectivity of the aldehyde groups with respect to the hydrogenation reaction was measured under the reaction condition 1 described below so as to calculate the selectivity of the hydrogenated compounds. The results are shown in Tab. 1 along with the corresponding chemical reaction formula (Formula 1). Note that, in examples and comparative examples described below, conversion rates of the substrates and selectivity of the products were measured through a gas chromatograph. GC-2014 made by Simazu was used as the gas chromatograph, and KOCL (3m) was used as a column filler.

### [Reaction condition 1]

- Catalyst quantity: 0.008 mmol in terms of the amount of silver through metal conversion
- Substrate: Citral (geranial:neral = 2:1) 0.25 mmol
- Solvent:Tetrahydrofuran (THF) 5 ml
- Hydrogen Pressure: 1.5 MPa
- Reaction Temperature: 150 °C
- Reaction Interval: Shown in Tab. 1

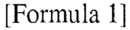

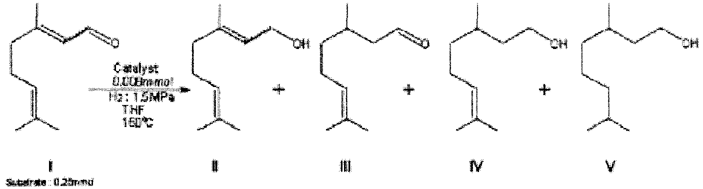

**[Tab. 1]**

| | Catalyst | Time (h) | Conversion rate of I (%) | Selectivity of II (%) | Selectivity of III (%) | Selectivity of IV (%) | Selectivity of V (%) |
|---|---|---|---|---|---|---|---|
| Example 1 | [AgNPs@CeO₂-D] | 12 | >99 | 98 | <1 | 2 | 0 |
| Comparative example 1 | [AgNPs@CeO₂] | 72 | >99 | 96 | 1 | 3 | 0 |

As shown in Tab. 1, the selective hydrogenation method using the selective hydrogenation catalyst (I) of the present invention, allows the reaction interval thereof to be significantly reduced as compared to that of the selective hydrogenation catalyst (i). The method also enhances the conversion rates of the chemical compounds containing hydrogenation reaction sites as the substrates and oxygen-containing polar functional groups as well as the selectivity of the selectively hydrogenated product intended.

### [Reusability of selective hydrogenation catalyst]

Further, the reusability of the selective hydrogenation catalyst was tested with respect to the selective hydrogenation catalyst (I) of Example 1 after being used for selective hydrogenation reaction. The catalyst was washed by tetrahydrofuran, dried and then underwent the chemical reaction under the condition identical with that described in Example 1 before being tested for catalyst reusability. The results are shown in Tab. 2. The reaction intervals are shown in Tab. 2.

**[Tab. 2]**

| | Number of repeated use | Time (h) | Conversion rate of I (%) | Selectivity of II (%) |
|---|---|---|---|---|
| Example 1 | 0 | 12 | >99 | 98 |
| Repeated use 1 | 1 | 12 | >99 | 96 |
| Repeated use 2 | 2 | 12 | >99 | 97 |

### [Examples 2 to 17]

Selective hydrogenation reaction was performed using the catalyst (I) of the example obtained through production example 1 with respect to substrates containing other aldehyde groups as is the case with Example 1. And, the conversion rate of the chemical compounds containing oxygen-containing polar functional groups and hydrogenation reaction sites, as the substrates, was tested as well as the selectivity of the selectively hydrogenated product intended. The results are summarized in Tabs. 3 and 4.

### [Reaction condition 2]

- Catalyst quantity:0.008 mmol in terms of the amount of silver through metal conversion
- Substrate: shown in Tabs. 3 and 4 (0.25 mmol each)
- Solvent:Tetrahydrofuran (THF) 5ml
- Hydrogen Pressure: 1.5 MPa
- Reaction Temperature: 150°C
- Reaction Interval: shown in Tabs. 3 and 4

**[Tab. 3]**

| | Substrates | Products | Time (h) | Conversion rates (%) | Selectivity |
|---|---|---|---|---|---|
| Example 2 | 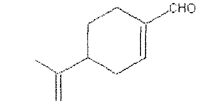 | 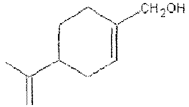 | 18 | 98 | 94 |
| Example 3 | 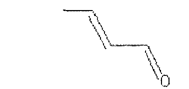 | 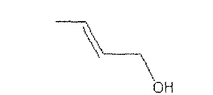 | 6 | 96 | 91 |
| Example 4 | 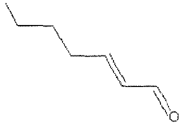 | 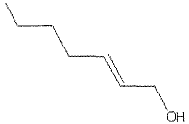 | 6 | 98 | 86 |
| Example 5 | 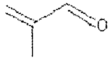 | 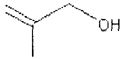 | 24 | 96 | 81 |
| Example 6 | 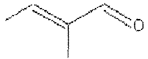 | 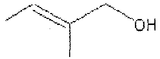 | 12 | 94 | 92 |
| Example 7 | 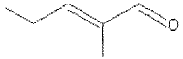 | 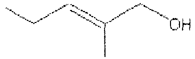 | 8 | 98 | 92 |
| Example 8 | 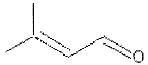 | 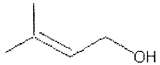 | 6 | 97 | 92 |
| Example 9 | 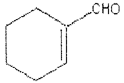 | 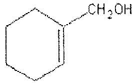 | 15 | 100 | 92 |
| Example 10 | 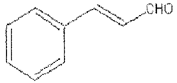 | 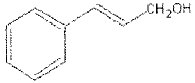 | 3 | 98 | 94 |

**[Tab. 4]**

| | Substrates | Products | Time (h) | Conversion rates (%) | Selectivity (%) |
|---|---|---|---|---|---|
| Example 11 | 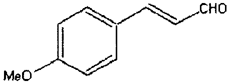 | 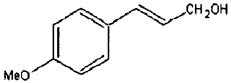 | 10 | 100 | 88 |
| Example 12 | 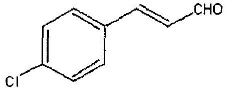 | 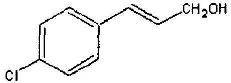 | 3 | 100 | 96 |
| Example 13 | 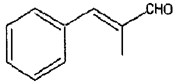 | 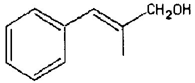 | 12 | 94 | 98 |
| Example 14 | 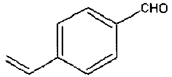 | 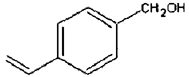 | 24 | 98 | 97 |
| Example 15 | 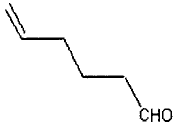 | 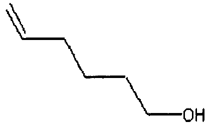 | 8 | 92 | 93 |
| Example 16 | 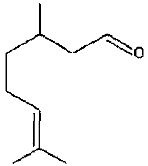 | 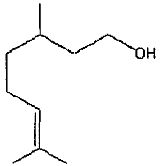 | 12 | >99 | >99 |
| Example 17 | 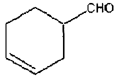 | 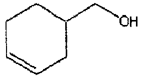 | 6 | 98 | 96 |

The above described results indicate that the selective hydrogenation catalyst of the present invention enables a superior selective hydrogenation reaction of chemical compounds containing various kinds of aldehyde groups as substrates and hydrogenation reaction sites such as carbon-carbon double bond.

### [Example 18 and Comparative examples 2 and 3]: Selective hydrogenation reaction of nitro groups.

Selectivities with respect to 3-nitrostyrene, a substrate containing nitro group, were measured using the catalyst (I) of example obtained through production example 1 and the catalyst (i) of comparative example obtained through production example 2 under the reaction condition 3 described below. The results are shown in Tab. 5 along with the corresponding chemical reaction formula (Formula 2).

### [Reaction condition 3]

- Catalyst quantity:0.008 mmol in terms of the amount of silver through metal conversion
- Substrate: 3-nitrostyrene 0.5 mmol
- Solvent: dodecane 5 ml
- Hydrogen Pressure: 0.6 MPa
- Reaction Temperature: 110°C
- Reaction Interval: shown in Tab. 5

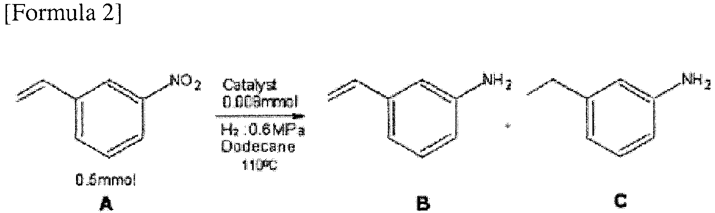

**[Tab. 5]**

| | Catalyst | Time (h) | Conversion rate of A (%) | Selectivity of B (%) | Selectivity of C (%) |
|---|---|---|---|---|---|
| Example 18 | [AgNPs@CeO₂-D] | 6 | 98 | 95 | <1 |
| Comparative example 2 | [AgNPs@CeO₂] | 6 | 26 | 23 | <1 |
| Comparative example 3 | [AgNPs@CeO₂] | 24 | 96 | 92 | <1 |

The above described results show that the catalyst, according to the present invention, allows 3-nitrostyrene, a substrate containing nitro group, to undergo a superior selective hydrogenation reaction.

### [Example 19, Comparative Examples 4 and 5]: Selective hydrogenation reaction of epoxide group

Selectivities with respect to styrene oxide, a substrate containing epoxy group, were measured as is the case with Example 1 using the catalyst (I) of example obtained through production example 1 and the catalyst (i) of comparative example obtained through production example 2 under the reaction condition 4 described below. The results are summarized in Tab. 6 along with the corresponding chemical reaction formula (Formula 3).

### [Reaction condition 4]

- Catalyst quantity: 0.008 mmol in terms of the amount of silver through metal conversion
- Substrate: styrene oxide 0.5 mmol
- Solvent: Toluene 5 ml
- Hydrogen Pressure: 0.6 MPa
- Reaction Temperature: 110°C
- Reaction Interval: shown in Tab. 6

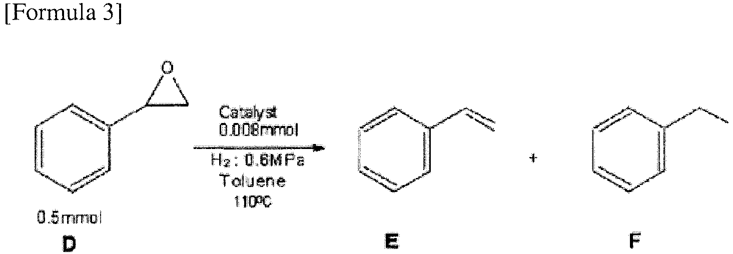

**[Tab. 6]**

| | Catalyst | Time (h) | Conversion rate of D (%) | Selectivity of E (%) | Selectivity of F (%) |
|---|---|---|---|---|---|
| Example 19 | [AgNPs@CeO₂-D] | 4 | 98 | 96 | <1 |
| Comparative example 4 | [AgNPs@CeO₂] | 4 | 21 | 18 | <1 |
| Comparative example 5 | [AgNPs@CeO₂] | 20 | 95 | 93 | <1 |

The above described results show that the catalyst, according to the present invention, allows styrene oxide, a substrate containing epoxy group, to undergo a superior selective hydrogenation reaction.

### [Examples 20 to 31]

The effects of adding amines, according to the present invention, were tested using catalyst (I) of example obtained through production example 1. The results are summarized in Tab. 7 along with its reaction conditions and corresponding chemical reaction formula (Formula 4).

### [Reaction condition 5]

- Catalyst quantity: 0.008 mmol in terms of the amount of silver through metal conversion
- Substrate: 3-vinyl benzaldehyde 0.25 mmol
- Solvent: Tetrahydrofuran (THF) 5ml
- Amines: shown in Tab. 7 with the amount used
- Hydrogen Pressure: 0.6 MPa
- Reaction Temperature: 110 °C
- Reaction Interval: shown in Tab. 7

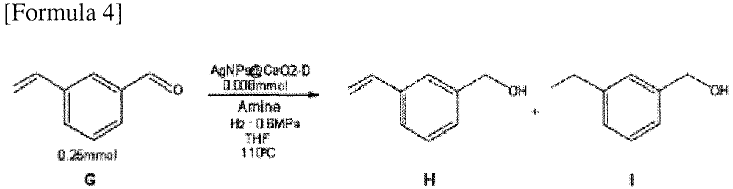

**[Tab. 7]**

| | Amines | Time (h) | Conversion rate of G (%) | Selectivity of H (%) | Selectivity of I (%) |
|---|---|---|---|---|---|
| Example 20 | Triethylamine 1mmol | 2 | 68 | >99 | <1 |
| Example 21 | Triethylamine 1mmol | 4 | >99 | >99 | <1 |
| Example 22 | Triethylamine 1mmol | 6 | >99 | >99 | <1 |
| Example 23 | Triethylamine 1mmol | 10 | >99 | 99 | 1 |
| Example 24 | Triethylamine 2.5mmol | 8 | 99 | >99 | <1 |
| Example 25 | Triethylamine 2.5mmol | 12 | 100 | 99 | 1 |
| Example 26 | Tributylamine 1mmol | 4 | 83 | >99 | <1 |
| Example 27 | Trihexylamine 1mmol | 4 | 42 | >99 | <1 |
| Example 28 | Trioctylamine 1mmol | 4 | 24 | 99 | 1 |
| Example 29 | Dibenzylamine 1mmol | 4 | 53 | 99 | 1 |
| Example 30 | None | 4 | 69 | 97 | 3 |
| Example 31 | None | 6 | 98 | 97 | 3 |

### [Examples 32 and 33]

The effects of adding amines, according to the present invention, were tested under the condition identical with the reaction condition 5 except that the substrate was changed into cinnamaldehyde. The results are summarized in Tab. 8 along with its reaction conditions and corresponding chemical reaction formula (Formula 5).

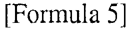

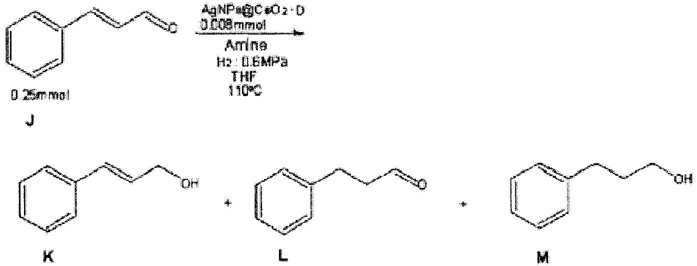

**[Tab. 8]**

| | Amines | Time (h) | Conversion rate of J (%) | Selectivity of K (%) | Selectivity of L (%) | Selectivity of M (%) |
|---|---|---|---|---|---|---|
| Example 32 | Triethylamine 2.5mmol | 10 | >99 | >98 | <1 | <1 |
| Example 33 | None | 10 | >99 | 85 | <1 | 15 |

As indicated above, it was found that by adding amines to the chemical reaction using the catalyst of the present invention, enhanced was the selectivity thereof even in the case where the substrate was changed. Moreover, it was found that the selectivities had been enhanced overall even though its effectiveness varied in accordance with the species of amines added.

### [Examples 34 to 36]

The effects of adding an inorganic base, according to the present invention, were tested using catalyst (I) of example obtained through production example 1. The results are summarized in Tab. 9 along with its reaction conditions and corresponding chemical reaction formula (Formula 6).

### [Reaction condition 6]

- Catalyst quantity: 0.008 mmol in terms of the amount of silver through metal conversion
- Substrate: 3-vinyl benzaldehyde 0.25 mmol
- Solvent: Tetrahydrofuran (THF) 5 ml
- Inorganic base: Cs₂CO₃ 1 mmol
- Hydrogen Pressure: 0.6 MPa
- Reaction Temperature: 110 °C
- Reaction Interval: shown in Tab. 9

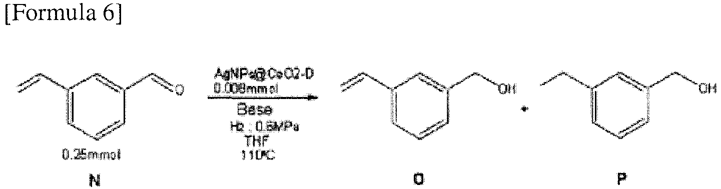

**[Tab. 9]**

| | Inorganic base | Time (h) | Conversion rate of N (%) | Selectivity of O (%) | Selectivity of P (%) |
|---|---|---|---|---|---|
| Example 34 | Cs₂CO₃ | 4 | >99 | >99 | <1 |
| Example 35 | None | 4 | 69 | 97 | 3 |
| Example 36 | None | 6 | 98 | 97 | 3 |

As indicated above, it was found that adding an inorganic base to the chemical reaction using the catalyst of the present invention had enhanced the conversion rate and selectivity thereof.

### [Examples 37 and 38]

The effects of adding an inorganic base, according to the present invention, were tested under the condition identical with the reaction condition 6 except that the substrate was changed to cinnamaldehyde. The results are summarized in Tab. 10 along with the corresponding chemical reaction formula (Formula 7).

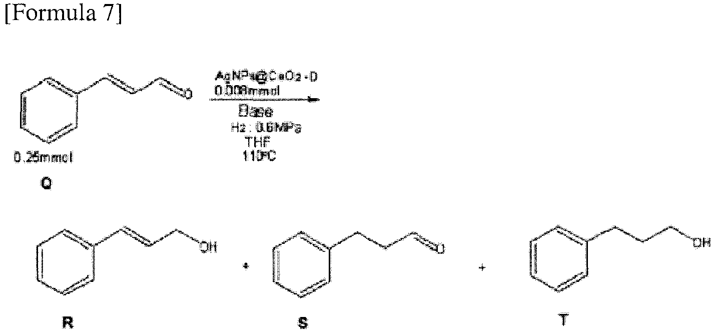

**[Tab. 10]**

| | Inorganic base | Time (h) | Conversion rate of Q (%) | Selectivity of R (%) | Selectivity of S (%) | Selectivity of T (%) |
|---|---|---|---|---|---|---|
| Example 37 | Cs₂CO₃ | 6 | >99 | 98 | <1 | 2 |
| Example 38 | None | 6 | 96 | 93 | 2 | 5 |

### [Examples 39 to 42]

Further, the effects of adding an inorganic base according to the present invention were tested under the condition identical with the reaction condition 6 except that the substrate was changed to the citral of example 1. The results are summarized in Tab. 11 along with the corresponding chemical reaction formula (Formula 8).

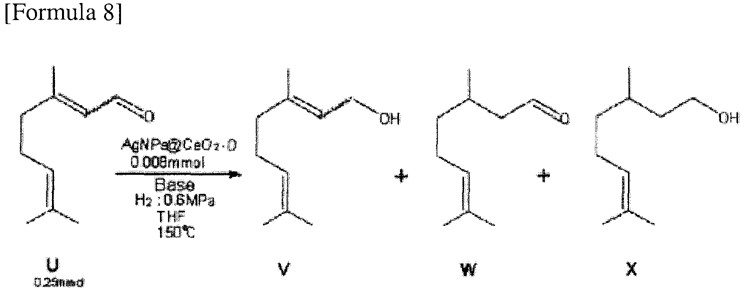

**[Tab. 11]**

| | Inorganic base | Time (h) | Conversion rate of U (%) | Selectivity of V (%) | Selectivity of W (%) | Selectivity of X (%) |
|---|---|---|---|---|---|---|
| Example 39 | Cs₂CO₃ | 12 | >99 | >99 | <1 | <1 |
| Example 40 | Cs₂CO₃ | 18 | >99 | 99 | <1 | 1 |
| Example 41 | None | 18 | >99 | 98 | <1 | 1 |
| Example 42 | None | 18 | >99 | 93 | <1 | 7 |

As indicated above, it was found that adding the inorganic base to the chemical reaction using the catalyst of the present invention leads to superior characteristics thereof regarding conversion rate and selectivity even in the case where the substrates were changed.

### [Examples 43 to 63]

Further, the effects of adding an inorganic base, according to the present invention, for other substrates were tested under the condition identical with the reaction condition 6. The results are summarized in Tabs. 12 to 14. Note that the symbols "(a)" through "(g)" in Tabs. 12 to 14, represent variations of each reaction conditions. The conditions can be summarized as follows. The conditions regarding reusability are identical with those of example 1.

### [Reaction condition: (a)]

- Catalyst quantity: 0.016 mmol in terms of the amount of silver through metal conversion
- Substrate: 0.25 mmol
- Solvent: Tetrahydrofuran (THF) 5 ml
- Inorganic base: Cs₂CO₃ 1 mmol
- Hydrogen Pressure: 0.6 MPa
- Reaction Temperature: 110°C
- Reaction Interval: shown in Tabs. 12 and 13.

### [Reaction condition :(b)]

- Catalyst quantity: 0.016 mmol in terms of the amount of silver through metal conversion
- Substrate: 0.25 mmol
- Solvent: Tetrahydrofuran (THF) 5 ml
- Inorganic base: Cs₂CO₃ 1 mmol
- Hydrogen Pressure: 1.5MPa
- Reaction Temperature: 60°C
- Reaction Interval: Shown in Tab. 12

### [Reaction condition : (c)]

- Catalyst quantity: 0.008 mmol in terms of the amount of silver through metal conversion
- Substrate: 0.25 mmol
- Solvent: Tetrahydrofuran (THF)5ml
- Inorganic base: Cs₂CO₃ 1 mmol
- Hydrogen Pressure: 1.5MPa
- Reaction Temperature: 150°C
- Reaction Interval: Shown in Tabs. 12 to 13

### [Reaction condition: (d)]

- Catalyst quantity: 0.016 mmol in terms of the amount of silver through metal conversion
- Substrate: 0.25 mmol
- Solvent: Tetrahydrofuran (THF) 5 ml
- Inorganic base: Rb₂CO₃ 1 mmol
- Hydrogen Pressure: 0.6 MPa
- Reaction Temperature: 110 °C
- Reaction Interval: Shown in Tab. 12

### [Reaction condition: (e)]

- Catalyst quantity: 0.016 mmol in terms of the amount of silver through metal conversion
- Substrate: 0.25 mmol
- Solvent: Tetrahydrofuran (THF)5 ml
- Inorganic base: Na₂CO₃ 1 mmol
- Hydrogen Pressure: 0.6MPa
- Reaction Temperature: 110°C
- Reaction Interval: Shown in Tab. 12

### [Reaction condition: (f)]

- Catalyst quantity: 0.016 mmol in terms of the amount of silver through metal conversion
- Substrate: 0.25 mmol
- Solvent: Tetrahydrofuran (THF) 5 ml
- Inorganic base: K₂CO₃ 1 mmol
- Hydrogen Pressure: 0.6 MPa
- Reaction Temperature: 110°C
- Reaction Interval: Shown in Tab. 12

### [Reaction condition: (g)]

- Catalyst quantity: 0.016 mmol in terms of the amount of silver through metal conversion
- Substrate: 0.25 mmol
- Solvent: Tetrahydrofuran (THF) 5 ml
- Inorganic base: Cs₂CO₃ 1 mmol
- Hydrogen Pressure: 0.6 MPa
- Reaction Temperature: 80°C
- Reaction Interval: Shown in Tab. 14

**[Tab. 12]**

| | Reaction conditions | Substrates | Products | Time (h) | Conversion rates (%) | Selectivity (%) |
|---|---|---|---|---|---|---|
| Example 43 | (a) | 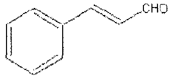 | 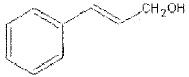 | 6 | >99 | >99 |
| Repeated use | (a) | 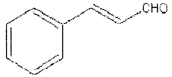 | 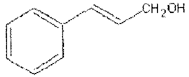 | 6 | >99 | >99 |
| Repeated use 2 | (a) | 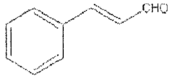 | 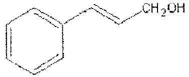 | 6 | >99 | >99 |
| Example 44 | (d) | 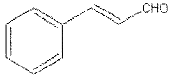 | 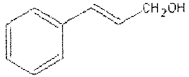 | 6 | 77 | 99 |
| Example 45 | (e) | 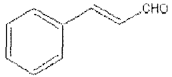 | 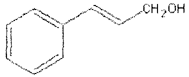 | 6 | 58 | 94 |
| Example 46 | (f) | 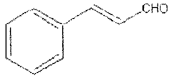 | 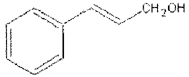 | 6 | 56 | 95 |
| Example 47 | (c) | 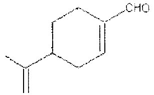 | 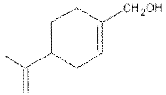 | 18 | >99 | 98 |
| Example 48 | (b) | 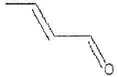 | 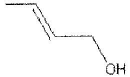 | 8 | >99 | 93 |
| Example 49 | (b) | 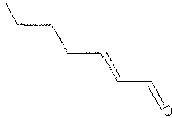 | 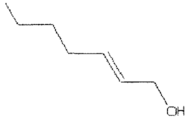 | 12 | 98 | 96 |
| Example 50 | (a) | 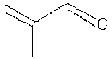 | 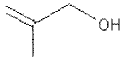 | 8 | 97 | 95 |
| Example 51 | (a) | 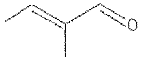 | 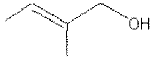 | 8 | 98 | 97 |

**[Tab. 13]**

| | Reaction conditions | Substrates | Products | Time (h) | Conversion rates (%) | Selectivity (%) |
|---|---|---|---|---|---|---|
| Example 52 | (c) | 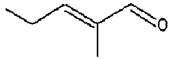 | 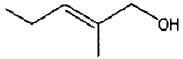 | 8 | >99 | 97 |
| Example 53 | (a) | 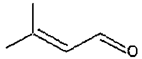 | 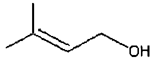 | 8 | >99 | 95 |
| Example 54 | (a) | 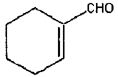 | 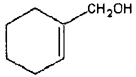 | 15 | 100 | 97 |
| Example 55 | (a) | 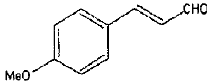 | 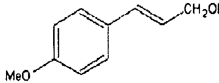 | 10 | >99 | 97 |
| Example 56 | (a) | 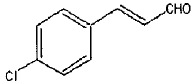 | 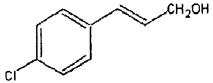 | 3 | >99 | >99 |
| Example 57 | (a) | 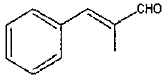 | 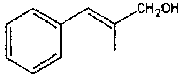 | 6 | >99 | >99 |
| Example 58 | (a) | 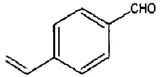 | 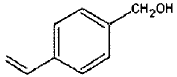 | 6 | >99 | >99 |
| Example 59 | (a) | 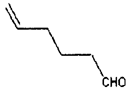 | 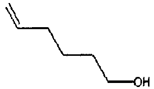 | 12 | >99 | 96 |
| Example 60 | (a) | 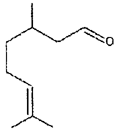 | 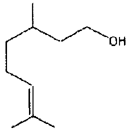 | 6 | 98 | >99 |
| Example 61 | (c) | 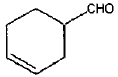 | 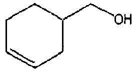 | 6 | >99 | 98 |

**[Tab. 14]**

| | Reaction conditions | Substrates | Products | Time (h) | Conversion rates (%) | Selectivity (%) |
|---|---|---|---|---|---|---|
| Example 62 | (g) | 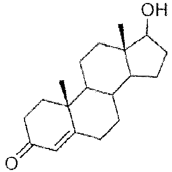 | 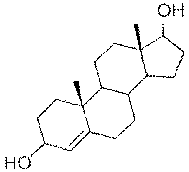 | 20 | 84 | 92 |
| Example 63 | (g) | 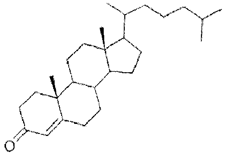 | 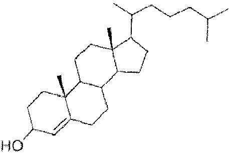 | 24 | 96 | 82 |

### [Production example 3] (Production of selective hydrogenation catalyst (II) of the examples using coprecipitation technique)

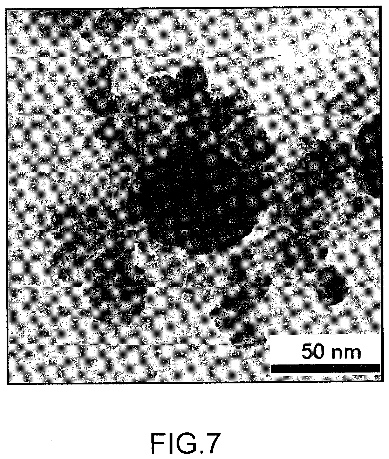

15 mmol of cesium hydroxide as a coprecipitation agent was dissolved into 100 ml of pure water to prepare a coprecipitation agent water solution. 10.0g of cerium dioxide (Reference catalyst JRC-CEO-3, BET specific surface area of 81 m²/g) as a cerium-containing oxide support was added into the coprecipitation agent water solution to suspend the cerium dioxide support. 1.5 mmol of silver nitrate and a mixed aqueous solution (raw material solution) containing 3 mmol of cerium nitrate (III) were added into said suspension to coprecipitate the silver compound and cerium compound on the cerium dioxide support. Then, a solution containing 2.2 mmol of cerium nitrate (III) was further added by drops to said suspension to further precipitate the cerium compound on the mixed particles of coprecipitated silver/cerium compound. Note that the mixture of the suspension and the cerium nitrate (III) water solution had a pH of 8.9. After filter washing the support containing cerium compound-supporting coprecipitation mixed particles obtained, the support was dried for 16 hours at room temperature under atmospheric pressure and then calcined for 4 hours at 400°C under atmospheric pressure, thereby obtaining a selective hydrogenation catalyst (II) with silver component-cerium oxide complex supported on the cerium dioxide containing oxide support. The amount of silver in the selective hydrogenation catalyst (II) thus obtained was 1.49 wt % through metal conversion. Also, the molar ratio of [Ag/CeO₂] was 0.29. FIG. 7 shows an entire STEM (Scanning Transmission Electron Microscope) image of the selective hydrogenation catalyst (II) of the example obtained through production example 3.

### [Production example 4] (Production of selective hydrogenation catalyst (III) of the example using coprecipitation technique)

The selective hydrogenation catalyst (III) with the silver component-cerium oxide complex supported on the cerium-containing oxide support was obtained in manner similar to the production example 3 except that: the amount of cesium hydroxide was 5 mmol; the amount of cerium nitrate in the mixed aqueous solution (raw material solution) of silver nitrate and cerium nitrate (III) was 0.75 mmol; and the amount of cerium nitrate in the cerium nitrate water solution was 0.59 mmol. Note that the mixture before filtering had a pH of 8.9. The amount of silver in the selective hydrogenation catalyst (III) obtained in this manner was 1.57wt% through metal silver conversion. Also, the molar ratio of [Ag/CeO₂] was 1.12.

### [Production example 5] (Production of selective hydrogenation catalyst (IV) of the example using coprecipitation technique)

The selective hydrogenation catalyst (IV) with the silver component-cerium oxide complex supported on the cerium-containing oxide support was obtained in manner similar to the production example 3 except that: the amount of cesium hydroxide was 20 mmol; and 10 g of cerium oxide (Anan Kasei Co., Ltd cerium oxide NT, BET specific surface area of 1.3m²/g) was used as a cerium-containing oxide support. Note that the mixture before filtering had a pH of 12.4. The amount of silver in the selective hydrogenation catalyst (IV) obtained in this manner was 1.49 wt% through metal silver conversion. Also, the molar ratio of [Ag/CeO₂] was 0.29.

### [Production example 6] (Production of selective hydrogenation catalyst (V) of the example through coprecipitation technique)

53 mmol of cesium hydroxide as a precipitating agent was dissolved into 100 ml of pure water to prepare coprecipitation agent water solution.

Further, 10.0 g of cerium dioxide (Anan Kasei Co., Ltd; cerium oxide NT; BET specific surface area of 1.3m²/g) as a cerium-containing oxide support was added into the coprecipitation agent water solution to suspend the cerium dioxide support. A mixed aqueous solution (raw material solution) containing 5 mmol of silver nitrate and 9 mmol of cerium nitrate (III) was added into said suspension. Then, a water solution containing 6.3 mmol of cerium nitrate (III) was further added by drops to said suspension. The mixture thus obtained had a pH of 12.0. After filter washing the support containing cerium compound-supporting coprecipitation mixed particles obtained, the support was dried for 16 hours at room temperature under atmospheric pressure and then calcined for 4 hours at 400°C (ambient pressure), thereby obtaining a selective hydrogenation catalyst (ii) with silver component-cerium oxide complex supported on the cerium containing oxide support. The amount of silver in the selective hydrogenation catalyst (ii) thus obtained was 4.29 wt % through metal conversion. Also, the molar ratio of [Ag/CeO₂] was 0.33.

### [Production example 7] (Production of selective hydrogenation catalyst (VI) of the example using coprecipitation technique)

A selective hydrogenation catalyst (iii) with silver component-cerium oxide complex supported on the cerium-containing oxide support was obtained in manner similar to the production example 6 except that a cerium dioxide (DAIICHI KIGENSO KAGAKU KOGYO CO., LTD cerium oxide LTT, BET specific surface area of 180m²/g) was used as a cerium-containing oxide support in production example 6. Note that the mixture before filtering had a pH of 12.0. The amount of silver of the selective hydrogenation catalyst (iii) obtained in this manner was 4.29 wt % through metal conversion. Also, the molar ratio of [Ag/CeO₂] was 0.33.

### [Examples 64 to 68]

Selective hydrogenation reaction was performed with respect to cinnamaldehyde as a substrate containing aldehyde group using the above described selective hydrogenation catalysts (II) to (VI), and selectivity of the aldehyde groups with respect to the hydrogenation reaction was measured so as to calculate the selectivity of the hydrogenated compounds under the reaction condition 7 described below. The results are shown in Tab. 15 along with the corresponding chemical reaction formula (Formula 9).

### [Reaction condition 7]

- Catalyst quantity: 2.0 g
- Substrate: cinnamaldehyde 7.5 mmol
- Solvent: tetrahydrofuran 50 ml
- Base: cesium carbonate 1 mmol
- Hydrogen Pressure: 0.6 MPa
- Reaction Temperature: 110°C
- Reaction Interval: 4 hours

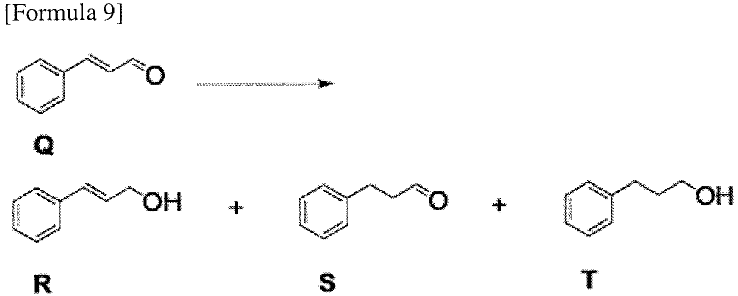

**[Tab. 15]**

| | Catalyst | Conversion rate of Q (%) | Yield of R (%) | Selectivity of R (%) | Yield of T (%) |
|---|---|---|---|---|---|
| Example 64 | (II) | 99.3 | 97.0 | 97.7 | 2.3 |
| Example 65 | (III) | 99.2 | 93. 2 | 94.0 | 5.9 |
| Example 66 | (IV) | 98.2 | 97.3 | 99.1 | 0.9 |
| Example 67 | (V) | 23.0 | 16.2 | 70.4 | 6.8 |
| Example 68 | (VI) | 85.3 | 50.8 | 59.6 | 34.5 |

The above results show that the selective hydrogenation catalysts, produced in accordance with the producing method using coprecipitation technique of the present invention, are further capable of performing superior selective hydrogenation reaction with respect to cinnamaldehyde, a substrate containing an aldehyde group.

## Claims

1. A selective hydrogenation catalyst **characterized in** comprising:
a metal oxide support; and
a silver component-cerium oxide complex that is supported on said metal oxide support and includes silver component particles as a core and cerium oxide particles as a shell layer supported on the surfaces of said silver component particles.

2. The selective hydrogenation catalyst according to claim 1, **characterized in that** a molar ratio of silver to said cerium oxide in said silver component-cerium oxide complex is 0.25 to 3.0, said molar ratio being calculated as a ratio of metallic silver in said silver component particles to said cerium oxide, in terms of metallic silver (Ag) / cerium oxide (CeO₂).

3. The selective hydrogenation catalyst according to claim 1 or claim 2, **characterized in that** said metal oxide support is a cerium-containing oxide.

4. The selective hydrogenation catalyst according to any one of claim 1 to claim 3, **characterized in that** said silver component-cerium oxide complex is formed into the shape of a true sphere.

5. A selective hydrogenation method for hydrogenating a compound having a hydrogenation reaction site and an oxygen-containing polar functional group, comprising a step of bringing said compound into contact with a hydrogen gas in a liquid phase under the presence of said catalyst of any one of claim 1 to claim 4.

6. A production method of a selective hydrogenation catalyst comprising a metal oxide support and a silver component-cerium oxide complex that is supported on said metal oxide support and includes silver component particles as a core and cerium oxide particles as a shell layer supported on the surfaces of said silver component particles, comprising:
a step of preparing a mixture by adding said metal oxide support to a liquid mixture of a reverse micelle containing said silver component particles and a reverse micelle containing said cerium oxide;
a step of drying said mixture to form a catalyst precursor with said silver component-cerium oxide complex being supported on said metal oxide support; and
a step of calcining said catalyst precursor.

7. A production method of a selective hydrogenation catalyst comprising a metal oxide support and a silver component-cerium oxide complex that is supported on said metal oxide support and includes silver component particles as a core and cerium oxide particles as a shell layer supported on the surfaces of said silver component particles, comprising:
a (i) step of preparing a raw material solution containing a mixed salt of a silver salt and a cerium salt;
a (ii) step of forming on said metal oxide support coprecipitation mixed particles of a silver compound and a cerium compound, by mixing said raw material solution and said metal oxide support under the presence of a coprecipitation agent acting on both silver and cerium ions;
a (iii) step of forming a catalyst precursor by adding a cerium salt to said coprecipitation mixed particles on said metal oxide support under the presence of said coprecipitation agent, and then allowing a precipitate of a cerium compound to be supported on said coprecipitation mixed particles; and
a (iv) step of calcining said catalyst precursor.

8. The selective hydrogenation catalyst production method according to claim 7, **characterized in that** said silver component is at least one of metal silver or silver oxide, and said cerium oxide is cerium dioxide.

9. The selective hydrogenation catalyst production method according to claim 7, **characterized in that** said coprecipitation mixed particles in said step (iii) are mixed particles of silver compound particles and cerium compound particles that are both hardly soluble to a solvent of said raw material solution.

10. The selective hydrogenation catalyst production method according to any one of claim 7 to claim 9, **characterized in that** said coprecipitation agent is an electrolyte containing negative ions capable of generating a hardly-soluble salt or a hardly-soluble compound with both silver ions and cerium ions as metal positive ions.

11. The selective hydrogenation catalyst production method according to any one of claim 7 to claim 10, **characterized in that** a suspension containing said metal oxide support is a suspension with cerium dioxide being suspended in a coprecipitation agent water solution.

12. The selective hydrogenation catalyst production method according to any one of claim 8 to claim 11, **characterized in that** said silver salt and said cerium salt in said raw material solution have common negative ions.

13. The selective hydrogenation catalyst production method according to claim 11, **characterized in that** a cerium ion precipitation in said step (iii) is coprecipitated on said metal oxide support.

14. The selective hydrogenation catalyst production method according to any one of claim 7 to claim 13, **characterized in that** a measured value of a specific surface area of said metal oxide support is 0.5 to 180 m²/g, when measured through a BET method.

## Patentansprüche

1. Selektiver Hydrierungskatalysator, **dadurch gekennzeichnet, dass** er umfasst: einen Metalloxidträger; und
einen Silberkomponenten-Ceroxid-Komplex, der auf dem Metalloxidträger getragen wird und Silberkomponentenpartikel als einen Kern und Ceroxidpartikel als eine Hüllenschicht umfasst, die auf den Oberflächen der Silberkomponentenpartikel getragen werden.

2. Selektiver Hydrierungskatalysator nach Anspruch 1, **dadurch gekennzeichnet, dass** ein Molverhältnis vom Silber zum Ceroxid in dem Silberkomponenten-Ceroxid-Komplex 0,25 bis 3,0 beträgt, wobei das Molverhältnis als Verhältnis von metallischem Silber in den Silberkomponentenpartikeln zum Ceroxid hinsichtlich metallischem Silber (Ag) / Ceroxid (CeO₂) berechnet wird.

3. Selektiver Hydrierungskatalysator nach Anspruch 1 oder Anspruch 2, **dadurch gekennzeichnet, dass** der Metalloxidträger ein Cer enthaltendes Oxid ist.

4. Selektiver Hydrierungskatalysator nach einem der Ansprüche 1 bis 3, **dadurch gekennzeichnet, dass** der Silberkomponenten-Ceroxid-Komplex in Form einer echten Kugel geformt wird.

5. Selektives Hydrierungsverfahren zum Hydrieren einer Verbindung mit einer Hydrierungsreaktionsstelle und einer sauerstoffhaltigen polaren funktionellen Gruppe, umfassend einen Schritt des Kontaktierens der Verbindung mit einem Wasserstoffgas in einer flüssigen Phase in Gegenwart des Katalysators nach einem der Ansprüche 1 bis 4.

6. Herstellungsverfahren für einen selektiven Hydrierungskatalysator, umfassend einen Metalloxidträger und einen Silberkomponenten-Ceroxid-Komplex, der auf dem Metalloxidträger getragen wird und Silberkomponentenpartikel als einen Kern und Ceroxidpartikel als eine Hüllenschicht umfasst, die auf den Oberflächen der Silberkomponentenpartikel getragen werden, umfassend:
einen Schritt der Herstellung eines Gemisches durch Zugabe des Metalloxidträgers zu einem flüssigen Gemisch aus einer Umkehrmizelle, die die Silberkomponentenpartikel enthält, und einer Umkehrmizelle, die das Ceroxid enthält;
einen Schritt des Trocknens der Mischung, um einen Katalysatorvorläufer zu bilden, wobei der Silberkomponenten-Ceroxid-Komplex auf dem Metalloxidträger getragen wird; und
einen Schritt des Kalzinierens des Katalysatorvorläufers.

7. Herstellungsverfahren für einen selektiven Hydrierungskatalysator, umfassend einen Metalloxidträger und einen Silberkomponenten-Ceroxid-Komplex, der auf dem Metalloxidträger getragen wird und Silberkomponentenpartikel als einen Kern und Ceroxidpartikel als eine Hüllenschicht umfasst, die auf den Oberflächen der Silberkomponentenpartikel getragen werden, umfassend:
einen (i) Schritt der Herstellung einer Rohmateriallösung, die ein gemischtes Salz eines Silbersalzes und eines Cersalzes enthält;
einen (ii) Schritt der Bildung auf dem Metalloxidträger durch Kopräzipitation gemischter Partikel einer Silberverbindung und einer Cerverbindung durch Mischen der Rohmateriallösung und des Metalloxidträgers in Gegenwart eines Kopräzipitationsmittels, das sowohl auf Silber- als auch auf Cerionen wirkt;
einen (iii) Schritt der Bildung eines Katalysatorvorläufers durch Zugabe eines Cersalzes zu den durch Kopräzipitation gemischten Partikeln auf dem Metalloxidträger in Gegenwart des Kopräzipitationsmittels und dann Zulassen, dass ein Präzipitat einer Cerverbindung auf den durch Kopräzipitation gemischten Partikeln getragen wird; und
einen (iv) Schritt des Kalzinierens des Katalysatorvorläufers.

8. Herstellungsverfahren für einen selektiven Hydrierungskatalysator nach Anspruch 7, **dadurch gekennzeichnet, dass** die Silberkomponente Metallsilber und/oder Silberoxid ist und das Ceroxid Cerdioxid ist.

9. Verfahren zur Herstellung eines selektiven Hydrierungskatalysators nach Anspruch 7, **dadurch gekennzeichnet, dass** die durch Kopräzipitation gemischten Partikeln in Schritt (iii) gemischte Partikel aus Silberverbindungspartikeln und Cerverbindungspartikeln sind, die beide durch ein Lösungsmittel der Rohmateriallösung kaum löslich sind.

10. Verfahren zur Herstellung eines selektiven Hydrierungskatalysators nach einem der Ansprüche 7 bis 9, **dadurch gekennzeichnet, dass** das Kopräzipitationsmittel ein Elektrolyt ist, der negative Ionen enthält, die ein schwerlösliches Salz oder eine schwerlösliche Verbindung mit sowohl Silberionen als auch Cerionen als positiv geladene Metallionen erzeugen können.

11. Verfahren zur Herstellung eines selektiven Hydrierungskatalysators nach einem der Ansprüche 7 bis 10, **dadurch gekennzeichnet, dass** eine Suspension, die den Metalloxidträger enthält, eine Suspension ist, in der Cerdioxid in einer wässrigen Kopräzipitationsmittellösung suspendiert ist.

12. Verfahren zur Herstellung eines selektiven Hydrierungskatalysators nach einem der Ansprüche 8 bis 11, **dadurch gekennzeichnet, dass** das Silbersalz und das Cersalz in der Rohmateriallösung gemeinsame negative Ionen aufweisen.

13. Verfahren zur Herstellung eines selektiven Hydrierungskatalysators nach Anspruch 11, **dadurch gekennzeichnet, dass** eine Cerionenpräzipitation in Schritt (iii) auf dem Metalloxidträger kopräzipitiert wird.

14. Verfahren zur Herstellung eines selektiven Hydrierungskatalysators nach einem der Ansprüche 7 bis 13, **dadurch gekennzeichnet, dass** ein gemessener Wert einer spezifischen Oberfläche des Metalloxidträgers 0,5 bis 180 m²/g beträgt, wenn er durch ein BET-Verfahren gemessen wird.

## Revendications

1. Catalyseur d'hydrogénation sélective **caractérisé en ce qu'**il comprend :
un support en oxyde métallique ; et
un complexe de composant d'argent et d'oxyde de cérium qui est supporté sur ledit support en oxyde métallique et comprend des particules de composant d'argent en tant que coeur et des particules d'oxyde de cérium en tant que couche de coque supportée sur les surfaces desdites particules de composant d'argent.

2. Catalyseur d'hydrogénation sélective selon la revendication 1, **caractérisé en ce qu'**un rapport molaire de l'argent audit oxyde de cérium dans ledit complexe de composant d'argent et d'oxyde de cérium est de 0,25 à 3,0, ledit rapport molaire étant calculé sous la forme d'un rapport de l'argent métallique dans lesdites particules de composant d'argent audit oxyde de cérium, en termes d'argent métallique (Ag) / oxyde de cérium (CeO₂).

3. Catalyseur d'hydrogénation sélective selon la revendication 1 ou la revendication 2, **caractérisé en ce que** ledit support en oxyde métallique est un oxyde contenant du cérium.

4. Catalyseur d'hydrogénation sélective selon l'une quelconque de la revendication 1 à la revendication 3, **caractérisé en ce que** ledit complexe de composant d'argent et d'oxyde de cérium est mis sous la forme d'une véritable sphère.

5. Procédé d'hydrogénation sélective pour hydrogéner un composé comportant un site de réaction d'hydrogénation et un groupe fonctionnel polaire contenant de l'oxygène, comprenant une étape de mise en contact dudit composé avec un gaz hydrogène dans une phase liquide en présence dudit catalyseur de l'une quelconque de la revendication 1 à la revendication 4.

6. Procédé de production d'un catalyseur d'hydrogénation sélective comprenant un support en oxyde métallique et un complexe de composant d'argent et d'oxyde de cérium qui est supporté sur ledit support en oxyde métallique et comprend des particules de composant d'argent en tant que coeur et des particules d'oxyde de cérium en tant que couche de coque supportée sur les surfaces desdites particules de composant d'argent, comprenant :
une étape de préparation d'un mélange en ajoutant ledit support en oxyde métallique à un mélange liquide d'une micelle inverse contenant lesdites particules de composant d'argent et d'une micelle inverse contenant ledit oxyde de cérium ;
une étape de séchage dudit mélange pour former un précurseur de catalyseur, ledit complexe de composant d'argent et d'oxyde de cérium étant supporté sur ledit support en oxyde métallique ; et
une étape de calcination dudit précurseur de catalyseur.

7. Procédé de production d'un catalyseur d'hydrogénation sélective comprenant un support en oxyde métallique et un complexe de composant d'argent et d'oxyde de cérium qui est supporté sur ledit support en oxyde métallique et comprend des particules de composant d'argent en tant que coeur et des particules d'oxyde de cérium en tant que couche de coque supportée sur les surfaces desdites particules de composant d'argent, comprenant :
une étape (i) de préparation d'une solution de matière première contenant un sel mixte d'un sel d'argent et d'un sel de cérium ;
une étape (ii) de formation sur ledit support en oxyde métallique de particules de coprécipitation mixtes d'un composé d'argent et d'un composé de cérium, en mélangeant ladite solution de matière première et ledit support en oxyde métallique en présence d'un agent de coprécipitation agissant à la fois sur les ions d'argent et de cérium ;
une étape (iii) de formation d'un précurseur de catalyseur en ajoutant un sel de cérium aux-dites particules de coprécipitation mixtes sur ledit support en oxyde métallique en présence dudit agent de coprécipitation, et en permettant ensuite à un précipité d'un composé de cérium d'être supporté sur lesdites particules de coprécipitation mixtes ; et
une étape (iv) de calcination dudit précurseur de catalyseur.

8. Procédé de production de catalyseur d'hydrogénation sélective selon la revendication 7, **caractérisé en ce que** ledit composant d'argent est au moins l'un du métal argent ou de l'oxyde d'argent, et ledit oxyde de cérium est du dioxyde de cérium.

9. Procédé de production de catalyseur d'hydrogénation sélective selon la revendication 7, **caractérisé en ce que** lesdites particules de coprécipitation mixtes dans ladite étape (iii) sont des particules mixtes de particules de composé d'argent et de particules de composé de cérium qui sont tant les unes que les autres peu solubles face à un solvant de ladite solution de matière première.

10. Procédé de production de catalyseur d'hydrogénation sélective selon l'une quelconque de la revendication 7 à la revendication 9, **caractérisé en ce que** ledit agent de coprécipitation est un électrolyte contenant des ions négatifs capables de générer un sel peu soluble ou un composé peu soluble avec tant des ions argent que des ions cérium en tant qu'ions métalliques positifs.

11. Procédé de production de catalyseur d'hydrogénation sélective selon l'une quelconque de la revendication 7 à la revendication 10, **caractérisé en ce qu'**une suspension contenant ledit support en oxyde métallique est une suspension avec du dioxyde de cérium en suspension dans une solution aqueuse d'agent de coprécipitation.

12. Procédé de production de catalyseur d'hydrogénation sélective selon l'une quelconque de la revendication 8 à la revendication 11, **caractérisé en ce que** ledit sel d'argent et ledit sel de cérium dans ladite solution de matière première comportent des ions communs négatifs.

13. Procédé de production de catalyseur d'hydrogénation sélective selon la revendication 11, **caractérisé en ce qu'**une précipitation d'ions cérium dans ladite étape (iii) est coprécipitée sur ledit support en oxyde métallique.

14. Procédé de production de catalyseur d'hydrogénation sélective selon l'une quelconque de la revendication 7 à la revendication 13, **caractérisé en ce qu'**une valeur mesurée d'une aire spécifique dudit support en oxyde métallique est de 0,5 à 180 m²/g, lorsqu'elle est mesurée par une méthode BET.
